(19) Europäisches Patentamt
European Patent Office
Office européen des brevets

(11) EP 0 728 209 B1

# (12) EUROPÄISCHE PATENTSCHRIFT

(45) Veröffentlichungstag und Bekanntmachung des Hinweises auf die Patenterteilung:
**11.01.2006 Patentblatt 2006/02**

(21) Anmeldenummer: **95900130.6**

(22) Anmeldetag: **10.11.1994**

(51) Int Cl.:
*C12N 15/57* (2006.01) *C12N 9/64* (2006.01)
*C07K 16/40* (2006.01) *G01N 33/573* (2006.01)
*A01K 67/027* (2006.01) *A61K 38/48* (2006.01)
*C12N 15/11* (2006.01) *A61K 31/70* (2006.01)

(86) Internationale Anmeldenummer:
**PCT/EP1994/003706**

(87) Internationale Veröffentlichungsnummer:
**WO 1995/014095 (26.05.1995 Gazette 1995/22)**

## (54) ENDOTHELINKONVERSIONSENZYM (ECE)

ENDOTHELIN-CONVERTING ENZYME

ENZYME DE CONVERSION DE L'ENDOTHELINE

(84) Benannte Vertragsstaaten:
**AT BE CH DE DK ES FR GB GR IE IT LI NL PT SE**

(30) Priorität: **16.11.1993 DE 4339100**
**07.02.1994 DE 4403665**
**12.04.1994 DE 4412372**

(43) Veröffentlichungstag der Anmeldung:
**28.08.1996 Patentblatt 1996/35**

(73) Patentinhaber: **Abbott GmbH & Co. KG**
**65205 Wiesbaden (DE)**

(72) Erfinder:
- **KRÖGER, Burkhard**
**D-67117 Limburgerhof (DE)**
- **SEULBERGER, Harald**
**D-69221 Dossenheim (DE)**
- **MEYER, Thomas**
**D-67346 Speyer (DE)**
- **SCHMIDT, Martin**
**D-64625 Bensheim (DE)**
- **JACOB, Elard**
**D-67304 Eisenberg (DE)**
- **OTTER, Rainer**
**D-69207 Sandhausen (DE)**
- **SUBKOWSKI, Thomas**
**D-67112 Mutterstadt (DE)**
- **HILLEN, Heinz**
**D-67454 Ha loch (DE)**

(74) Vertreter: **Grünecker, Kinkeldey,**
**Stockmair & Schwanhäusser**
**Anwaltssozietät**
**Maximilianstrasse 58**
**80538 München (DE)**

(56) Entgegenhaltungen:
**WO-A-92/13944**

- **JOURNAL OF BIOLOGICAL CHEMISTRY., Bd.268, Nr.28, 5. Oktober 1993, BALTIMORE US Seiten 21394 - 21398 M. TAKAHASHI ET AL 'Purification and characterization of endothelin-converting enzyme from rat lung' in der Anmeldung erwähnt**
- **DATABASE WPI Section Ch, Week 9236, Derwent Publications Ltd., London, GB; Class B04, AN 92-304620 & JP,A,4 210 593 (NISSHIN FLOUR MILLING CO) 31. Juli 1992**
- **FASEB JOURNAL, Bd.6, 1992 Seiten 2653 - 2658 T. OPGENORTH ET AL 'Endothelin-converting enzymes' in der Anmeldung erwähnt**
- **BIOSCIENCE, BIOTECHNOLOGY AND BIOCHEMISTRY, Bd.57, Nr.11, November 1993 Seiten 1944 - 1945 K.ARAI ET AL 'Aspergillomarasmine A and B, potent microbial inhibitors of endothelin-converting enzyme'**
- **BIOORGANIC & MEDICAL CHEMISTRY LETTERS, Bd.3, Nr.10, 1993 Seiten 1953 - 1958 S. BERTENSHAW ET AL 'Thiol and hydroxamic acid containing inhibitors of endothelin converting enzyme'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- BIOCHEMICAL AND BIOPHYSICAL RESEARCH COMMUNICATIONS., Bd.171, Nr.3, 1990, DULUTH, MINNESOTA US Seiten 1192 - 1198 K. OKADA ET AL 'Conversion of big endothelin-1 by membrane-bound metalloendopeptidase in cultured bovine endothelial cells' in der Anmeldung erwähnt
- PHARMACEUTICAL RESEARCH, Bd.5, Nr.9, 1988 Seiten 539 - 549 G. ZON 'Oligonucleotide analogues as potental chemotherapeutic agents'
- BIOCHEMICAL AND BIOPHYSICAL RESEARCH COMMUNICATIONS., Bd.203, Nr.3, 30. September 1994, DULUTH, MINNESOTA US Seiten 1417 - 1422 T. IKURA ET AL 'cDNA cloning and expression of bovine endothelin converting enzyme'
- JOURNAL OF BIOLOGICAL CHEMISTRY., Bd.269, Nr.28, 15. Juli 1994, BALTIMORE US Seiten 18275 - 19278 K. SHIMADA 'Cloning and functional expression of endothelin-converting enzyme from rat endothelial cells'
- JOURNAL OF BIOLOGICAL CHEMISTRY. (MICROFILMS), Bd.268, Nr.35, 15. Dezember 1993, BALTIMORE, MD US Seiten 26759 - 26766 K. OHNAKA ET AL 'Purification and characterization of a phosphoramidon-sensitive endothelin-converting enzyme in porcine aortic endothelium'

## Beschreibung

Die vorliegende Erfindung betrifft Endothelinkonversionsenzyme, Verfahren zu ihrer Herstellung und ihre Verwendung.

Erhöhte Endothelinspiegel werden mit einer Vielzahl von Erkrankungen wie essentielle Hypertonie, Herzinfarkt, akutes Nierenversagen, Schock oder Herzversagen in Zusammenhang gebracht. Auf einen solchen Zusammenhang lassen auch mit Endothelin-Antikörpern erzielbare Ergebnisse schließen. In Tiermodellen konnte damit die Größe eines Herzinfarktes dosisabhängig verringert (Watanabe et al., Nature 344, 114 (1990)), die Nierenfunktion positiv beeinflußt (Kon et al., J. Clin. Invest. 83, 1762 (1989)), und die Nephrotoxizität von Cyclosporin herabgesetzt werden (Kon et al., Kidney Int. 37, 1487 (1990)).

Endothelinkonversionsenzym (ECE-1) setzt Endothelin 1 aus dem 38 Aminosäuren bestehenden Vorläufermolekül Big-Endothelin-1 frei. Den von Endothelin-1 hervorgerufenen unerwünschten biologischen Effekten läßt sich z.B. durch Inhibierung des Endothelinkonversionsenzyms und damit der Endothelin-1 Biosynthese entgegenwirken.

Es sind Enzymaktivitäten, die Endotheline bzw. endothelinähnliche Moleküle aus den entsprechenden Vorläufermolekülen, den Big-Endothelinen freisetzen, aus verschiedenen Zellinien isoliert worden (Takeda et al., Biochem. Biophys. Res. Comm. 176, 860 (1991), Ohnaka et al., Biochem. Biophys. Res. Comm. 168, 1128 (1990); Matsumura et al. FEBS Lett. 293, 45 (1992), Ahn et al. Proc. Natl. Acad. Sci. USA 89, 8606 (1992), PCT WO 92/13944, Ohnaka et al. Clin. Exp. Hypertension A 14; Nr. 4 (1992), Okada et al. Biochem. Biophys. Res. Comm. 180, 1019 (1991), Takada et al. Biochem. Biophys. Res. Comm. 182, 1383 (1992), Opgenorth et al. FASEB 6, 2653 (1992)).

Diese Enzymaktivitäten sind jedoch bisher unzureichend charakterisiert worden. Sie liegen als wenig angereicherte Enzymmischungen vor. Solche unreinen Enzymmischungen sind jedoch für die Verwendung in Testverfahren zum Auffinden von spezifischen ECE-Inhibitoren schlecht geeignet, da andere, physiologisch nicht relevante Fremdproteasen den Enzymtest erheblich stören.

So wird Big-Endothelin beispielsweise auch durch die Serinprotease Chymothrypsin sowie durch Papain, Thermolysin und NEP 24.11 zu Endothelin und endothelinähnlichen Produkten gespalten, die in Testverfahren fälschlicherweise dem Enzym ECE zugeordnet werden. Infolgedessen ist die Beschreibung des Endothelinkonversionsenzyms in der Literatur widersprüchlich.

Die Angaben über das Molekulargewicht des Endothelinkonversionsenzyms variieren von 65 KDalton bis 540 KDalton; die Angaben der Km und vmax Werte weichen ebenfalls erheblich voneinander ab (Opgenorth et al. FASEB 6, 2653 (1992); Sawamura et a1. Biochem. Biophys. Acta 1161,295 (1993)). Auch gibt es Widersprüche darüber, ob das Endothelinkonversionsenzym in die Familie der Aspartatproteasen oder der Metalloproteasen einzuordnen ist (Sawamura et al. Biochem. Biophys. Acta 1161,295 (1993); Biochem. Pharmacol. 43,845 (1992). Ferner finden sich in der Literatur widersprüchliche Aussagen über charakteristische Angaben, ob es sich bei der Metalloprotease um ein cytoplasmatisches oder membrangebundenes Enzym handelt und welche Substrate von dem jeweiligen ECE umgesetzt werden (Big-Et-1; Big-Et-3): Matsumura et al. (FEBS Lett. 293, 45 (1992)): Takahashi et al. (J. Biol. Chem. 268;21394 (1993)); Okada et al. (Biochem. Biophys. Res. Comm. 180, 1019 (1991)); Ohnaka et al. (Clin. Exp. Hypertension A 14; Nr. 4 (1992)); Matsumura et al. (FEBS Lett. 305;86 (1992)).

Botehlo et al (Patentanmeldung WO 92/13944) beschreiben die Isolation von Endothelinkonversionsenzym aus humanem Lungengewebe. Die Reinigungsschritte ergaben eine Mischung von Proteinen verschiedener Molekulargewichte (zwischen 232 und 440 kDa), die Endothelinkonversionsenzym-Aktivität aufwiesen.

Die japanische Patentanmeldung JP04210593 beschreibt die Isolation von Endothelinkonversionsenzym u.a. aus humanem Plazentagewebe. Nach der Reinigung wurden Proteine mit Molekulargewichten von 240 und 500 kDa erhalten, die Endothelinkonversionsenzym-Aktivität aufwiesen.

Bisher wurde keine eindeutige Beschreibung des ECE gezeigt, die es ermöglicht, ECE einwandfrei zu definieren. Eine solche Definition kann möglicherweise erst durch das Bestimmen der Primärstruktur, der Aminosäuresequenz, des ECE erfolgen. Hierzu ist es notwendig, ECE zunächst in reiner Form herzustellen.

Es bestand daher die Aufgabe, Endothelinkonversionsenzym in reiner Form bereitzustellen.

Es wurde ein Endothelinkonversionsenzym, gekennzeichnet durch ein Molekulargewicht von 250 000 Dalton und die Aminosäurepartialsequenz SEQ ID NO: 1 gefunden.

Das erfindungsgemäße Endothelinkonversionsenzym weist folgende Merkmale auf: Es besitzt ein mittels SDS-Polyacrylamid-Gelelektrophorese unter nicht-reduzierenden Bedingungen ermitteltes Molekulargewicht von etwa 250 000 Dalton.

Unter reduzierenden Bedingungen zeigt es in der SDS-Polyacrylamidgelelektrophorese eine Bande von 125 000 Dalton; es besteht vermutlich aus einem Homodimer.

Das Endothelinkonversionsenzym ist glykosyliert. Die enzymatische Entfernung der zuckerreste bewirkt eine Veränderung des apparenten Molekulargewichts von 125 000 Dalton auf etwa 82 000 Dalton.

Die Sequenzierung von tryptischen Peptiden des Endothelinkonversionsenzyms liefert folgende Aminosäurepartialsequenzen SEQ ID NO: 1, 2, 3, 4, 5, 6.

Eine weitere Charakterisierung des erfindungsgemäßen Endothelinkonversionsenzyms ist in den Beispielen vorgenommen.

Ein weiterer Gegenstand der Erfindung sind Endothelinkonversionsenzyme, gekennzeichnet durch ein Molekulargewicht von etwa 250 000 Dalton.

Solche Endothelinkonversionsenzyme lassen sich aus anderen Organismen als Rind, beispielsweise aus menschlichen Zellen isolieren.

Die Erfindung betrifft weiterhin Endothelinkonversionsenzyme, die die in SEQ ID NO: 36 beschriebene Polypeptidsequenz enthalten.

Unter funktionellen Fragmenten sind solche Teilsequenzen zu verstehen, die noch die enzymatische Aktivität des Endothelinkonversionsenzyms, die antigenen Eigenschaften oder die Affinität zu Liganden, beispielsweise Bindeproteinen, besitzen.

Weitere funktionelle Fragmente sind solche Polypeptidsequenzen, die ausgehend von NO: 36 durch Insertion, Deletion oder Substitution von Aminosäuren oder Peptiden erhältlich sind, und die noch im wesentlichen die enzymatische Aktivität der Endothelinkonversionsenzyme besitzen und/oder mit den Endothelinkonversionsenzymen der Formel NO: 36 immunologisch kreuzreagieren.

Ein weiterer Gegenstand der Erfindung sind DNA-Sequenzen, die für ein Endothelinkonversionsenzym codieren und die aus der Gruppe, die von

a) DNA-Sequenzen mit der in SEQ ID NO: 35 beschriebenen Struktur,

b) DNA-sequenzen, die für Proteine mit der in SEQ ID NO: 36 beschriebenen Struktur codieren,

ausgewählt sind.

Unter Standardbedingungen sind beispielsweise Temperaturen zwischen 42 und 58°C in einer wäßrigen Pufferlösung mit einer Konzentration zwischen 0,1 und 1 x SSC (1 x SSC: 0,15M NaCl, 15mM Natriumcitrat pH 7,2) zu verstehen. Die experimentellen Bedingungen für DNA-Hybridisierung sind in Lehrbüchern der Gentechnik, beispielsweise in Sambrook et al., "Molecular Cloning", Cold Spring Harbor Laboratory, 1989, beschrieben.

Die Herstellung solcher DNA Sequenzen ist in Beispiel 8 beschrieben. DNA Sequenzen, deren Proteinprodukte von Antikörpern erkannt werden, die gegen die Proteine der SEQ ID NO: 30 oder 36 oder Fragmenten davon generiert wurden, lassen sich nach gut beschriebenen Methoden gewinnen.

Die Erzeugung von Antikörpern gegen solche Proteine ist z.B. beschrieben in Hudson, L. u. Hay, F.C., "Practical Immunology", Blackwell Sci. Pub., Oxford, 1980.

Die Verwendung von Antikörpern zur Auffindung von cENA-Sequenzen, die mit diesen Antikörpern kreuzreagierende Proteine kodieren, ist beispielsweise beschrieben in: "DNA Cloning Vol. I", Glover, D.M., Ed., IRL Press Oxford, 1985.

Die Erfindung betrifft weiterhin Verfahren zur Herstellung der Endothelinkonversionsenzyme, die dadurch gekennzeichnet sind, daß Säugerzellen, bevorzugt Endothelzellen, mit einem Inhibitor des Endothelinkonversionsenzyms zur Endothelinkonversionsenzym-Oberproduktion stimuliert werden und aus diesen Zellen Endothelinkonversionsenzym mit üblichen proteinchemischen Maßnahmen isoliert wird.

Für die Induktion des ECE in Säugerzellen können alle ECE-Inhibitoren Verwendung finden. Hierfür geeignete ECE-Inhibitoren sind z.B. beschrieben in JP-146737; JP05148277-A1; Jpn J. Biochem. 64; (8) Abst. 2367 (1992); Derwent NCE-93-0956 (1993); Derwent NCE-93-0971 (1993); J. Med. Chem. 36,173 (1993); EP 518299-A2. vorzugsweise findet Phosphoramidon Verwendung. Stimuliert werden die Zellen in Kultur durch Zugabe dieser Inhibitoren zum Zellkulturmedium für 6 Stunden bis zu 6 Tagen. Vorzugsweise wird die Stimulierung jedoch über 2-3 Tage durchgeführt. Die angewandten Inhibitorkonzentrationen betragen $10^{-2}$ M bis $10^{-7}$ M, vorzugsweise $10^{-3}$ M bis $10^{-4}$ M.

Die Herstellung der erfindungsgemäßen Endothelinkonversionsenzyme ist jedoch auch auf gentechnischem Weg möglich. Aufgrund der beschriebenen Aminosäurepartialsequenzen lassen sich synthetische Oligonukleotide, die diese Sequenzen codieren oder komplementär zu dafür codierenden Sequenzen sind, herstellen. Diese Oligonukleotide können als Hybridisierungsprobe zur Isolierung der entsprechenden Gene oder der cDNA-Moleküle aus Genpools wie Genbanken oder cDNA-Pools verwendet werden. Diese Art der Genisolierung ist dem Fachmann aus Lehrbtichern der Molekularbiologie wie Sambrook, Fritsch, Maniatis; Molecular Cloning, Cold Spring Harbor Laboratory Press, 1989, USA bekannt.

Mit Hilfe geeigneter synthetischer Oligonukleotide und der Polymerase-Chain-Reaction (PCR) können ebenfalls Teile des Gens beispielsweise aus cDNA-Banken oder Erststrang cDNA erhalten werden (PCR-Protocols; Academic Press; San Diego; USA; 1990). Oligonukleotide, die hierfür geeignet sind lassen sich aus den unter Seq ID No. 1 bis No. 6 beschriebenen Peptiden herleiten durch Übersetzen der Aminosäuresequenz in die doppelsträngige DNA-Sequenz (mit sense und antisense). Hierbei können aufgrund des degenerierten genetischen Codes degenerierte Oligonukleotidgemische aus einem Peptid abgeleitet werden und als Primer Verwendung finden. Die Oligonukleotide können aus den Peptiden auch unter Berücksichtigung der sogenannten "codon usage"; des preferentiellen Gebrauchs in einer Spezies, in diesem Fall Rind, von bestimmten Nukleotid-Tripletts für eine Aminosäure, abgeleitet werden. Die PCR wird dann mit zwei verschiedenen Oligonukleotiden als Primern durchgeführt, wobei die für die PCR notwendigen zwei Primer sich aus Strang (sense) und Gegenstrang (antisense) der Oligonukleotide zusammensetzen können. Ebenso Verwendung finden können Oligonukleotide, die von Teilen des cDNA-vektors hergeleitet sind, oder oligo-dT für den 3'Teil von mRNA.

Diese Genteile können dann wiederum eingesetzt werden, um mit dem Verfahren der Genisolierung das komplette Gen oder die vollstandige cDNA zu erhalten.

Auf diese Art lassen sich nicht nur Gene für die Endothelinkonversionsenzyme, die exakt die in SEQ ID NO: 1 beschriebene Aminosäurepartialsequenz besitzen, isolieren, sondern auch Gene für Varianten bzw. Endothelinkonversionsenzyme aus anderen Organismen, beispielsweise dem Menschen, die mindestens 80 % Homologie auf der Proteinebene zu SEQ ID NO: 1 aufweisen.

Die erfindungsgemäßen Endothelinkonversionsenzyme lassen sich besonders gut herstellen, indem man die entsprechenden Gene isoliert und diese Gene in einem Wirtsorganismus mit üblichen gentechnischen Methoden exprimiert.

Die erfindungsgemäßen ECE oder davon abgeleitete Peptide können verwendet werden zur Herstellung von Antikörpern oder Antikörperfragmenten wie z.B. $F_v$-Fragmenten, die beispielsweise zur Bestimmung von ECE, in Testverfahren zur Identifizierung von ECE-Hemmern oder als ECE-inhibitoren eingesetzt werden können.

Solche Antikörper sind generell in der Diagnostik von Erkrankungen, an denen Endothelinkonversionsenzyme beteiligt sind, wertvolle Hilfsmittel.

Für diese Zwecke können auch PCR-Primer, die sich von den erfindungsgemäßen DNA-Sequenzen ableiten, verwendet werden, um beispielsweise die ECE-Transkription zu messen oder den Genotyp zu bestimmen.

Mit Hilfe der erhaltenen Gene können in einer dem Fachmann bekannten Art und Weise nicht-menschliche Tiere in ihrem Gengut so verändert werden, daß sie ein oder mehrere Extrakopien dieses Gens erhalten oder daß das normale ECE-Gen ausgeschaltet wird. Diese transgenen Tiere eignen sich u.a. für Versuchszwecke.

Die Endothelinkonversionsenzyme, selbst eignen sich auch als Wirkstoff zur Herstellung von Arzneimitteln, beispielsweise bei Erkrankungen, bei denen erhöhte Spiegel von ECE-Substraten wie z.B. Bradykinin, Substanz P, Somatostatin, Neuropeptid Y vorliegen. Solche Erkrankungen sind beispielsweise Entzündungen, Asthma, Migräne, Rheuma, zelluläre Prozesse und Zellproliferation, die unter Beteiligung von Peptiden als Wachstumsfaktoren ablaufen. Anwendung finden kann ECE als Arzneimittel auch bei Krankheiten, in denen eine Vasodilatation vorliegt, die durch Verabreichen von ECE beseitigt werden kann, z.B. Septischer Schock, Migräne, Potenzstörungen.

Für eine Anwendung von ECE als Arzneimittel kann es unter Umständen notwendig sein, Teile des ECE mutativ zu verändern. Diese mutativen Veränderungen können beispielsweise zu einem ECE-Derivat führen, das nicht glykosyliert ist oder welches keinen Membrananker enthält (lösliches ECE). Ein solches lösliches ECE, welches keinen Membrananker mehr enthält, kann beispielsweise durch die Expression eines ECE-Fragmentes, welcher eine extrazelluläre, katalytisch wirksame Domäne enthält, hergestellt werden. Solche ECE-Fragmente kann man z.B. gewinnen, indem man die kodierende Sequenz zwischen Aminosäure 20 und 703 aus SEQ ID NO: 25, vorzugsweise zwischen Aminosäure 27 und 703 aus SEP ID NO: 25 mit der DNA Sequenz eines in vivo abspaltbaren Signalpeptid verknüpft und in einem, dem Fachmann bekannten, geeigneten Expressionsvektor in Eukaryontenzellen bringt. Als Signalpeptid eignet sich z.B. die Sequenz des humanen "Tissue Plasminogen Activator" zwischen Aminosäure Position -35 und -4 (Collen, D., Nature 301, 214 bis 221 (1983)). Veränderungen können dabei beispielsweise die Spezifität, die Wirkdauer, die Aufnahme beeinflussen. Ferner können funktionelle Teile von ECE mit Teilen anderer Proteine gekoppelt werden, um neue Proteine zu erzeugen, die als Arzneimittel Anwendung finden. Verändert werden kann ECE auch durch kovalente Modifizierung mit nicht-peptidischen Molekülen wie z.B. PEG, Dextran, Fettsäuren.

Die erfindungsgemäßen DNA-Sequenzen eignen sich auch zur Verwendung in der Gentherapie, beispielsweise zur Herstellung von gentherapeutischen Medikamenten. Krankheiten mit erhöhten Endothelin-Werten können auch mit von der ECE-DNA-Sequenz abgeleiteten Oligonukleotiden behandelt werden. Diese Oligonukleotide sind aus dem nicht-kodierenden DNA-Strang (anti-sense) abgeleitet und können als Arzneimittel angewandt werden. Eine hierauf basierende anti-sense-Therapie benutzt vorzugsweise von den Oligonukleotiden abgeleitete stabilere chemische Derivate. Beispielsweise können anti-sense-Oligonukleotide benutzt werden, die vorzugsweise aus dem Bereich, der durch die Positionen 31 bis 100 Seq ID 35 definiert ist, synthetisiert werden. Die anti-sense-Oligonukleotide haben vorzugsweise eine Länge von 15 bis 30 Resten. Anwendung findet die anti-sense-Therapie oder Prävention von Krankheiten in den gleichen Indikationen wie ECE-Inhibitoren, z.B. cerebralen Ischämien; Subarrachnoidalblutungen; Vasospasmen; koronaren Ischämien; Erkrankungen, die mit Zellproliferationsprozessen einhergehen wie Prostatahyperplasie, wie Atherosklerose, wie Restenose; Asthma; Hypertension; pulmonalem-Bluthochdruck; Organversagen wie Herzversagen und Nierenversagen; Nierenischämien; Neprotoxizität; Myokardinfarkt; koronarer Herzkrankheit; Sepsis.

Aus der DNA-Sequenz des ECE können auch Sequenzen abgeleitet werden, mit denen katalytische RNA-Moleküle erzeugt werden können, sogenannte "ribozymes". Diese katalytischen RNA-Moleküle wirken, indem sie nach Applikation die ECE-RNA oder ECE-DNA spalten oder inaktivieren und dadurch die Proteinsynthese des ECE verhindern.

Die ECE-DNA-Sequenzen können benutzt werden, um mit geeigneten Expressionsvektoren Zellen zu etablieren, die dieses Enzym exprimieren. Diese Zellen finden Verwendung u.a. als Arzneimittel.

Bevorzugt finden die Endothelinkonversionsenzyme in Testverfahren zur Identifizierung von ECE-Hemmstoffen Verwendung. Diese Testverfahren sind in der Regel enzymatische Reaktionen, bei denen die ECE-katalysierte Spaltung eines Substrates in Gegenwart von potentiellen Hemmstoffen gemessen wird.

Ein weiterer Gegenstand der Erfindung ist die Verwendung von Endothelinkonversionsenzym zur Identifizierung Von Hemmstoffen des Endothelinkonversionsenzyms. Die Hemmstoffe können mit einem Enzymtest unter Verwendung des erfindungsgemäßen Endothelinkonversionsenzyms identifiziert werden.

Zur Erfindung gehört auch die Verwendung von Endothelinkonversionsenzym zur Herstellung von Arzneimitteln, die ein Endothelinkonversionsenzym inhibieren. Dazu können bekannte chemische Verbindungen in einem Enzymtest unter Verwendung des erfindungsgemäßen Endothelinkonversionsenzyms eingesetzt werden und solche mit inhibierender Wirkung identifiziert werden. Diese Verbindung kann dann mit üblichen Träger- und Hilfsstoffen als Arzneimittel formuliert werden.

Beispiel 1

Stimulation des ECE-1 in primären Rinderendothelzellen FBHE durch Phosphoramidon

3 Röhrchen mit eingefrorenen FBHE-Rinderendothelzellen (ATCC CRL 1395) wurden in der 14. Passage aufgetaut und in 3 T175-Zellkulturflaschen (Fa. Sarstedt) mit je 40 ml Wachstumsmedium DMEM (Gibco Nr. 041-01965)
+ 5 ml/500 ml Glutamin (200 mM; Gibco 043-05030)
+ 1 ml/500 ml Gentamycin (Gibco 043-05750)
+ Non-Essentielle Aminosäuren; Endkonzentration: 1 x (Gibco Nr. 043-01140)
+ 10 % FCS (Gibco Nr. 011-06290; inaktiviert für 37 Minuten bei 56°C) + 25 ng/ml Basischer FGF (Intergen 4125-80)) gegeben. Diese Zellen wurden nach Standardverfahren bei 37°C; in einer Atmosphäre aus 7 % $CO_2$; 100 % Luftfeuchtigkeit inkubiert. Am darauffolgenden Tag erfolgte ein Mediumswechsel. Bei Erreichen der Konfluenz - in diesem Fall nach 4 Tagen - wurden die Zellen nach Standardverfahren durch Trypsinbehandlung passagiert und in 9 T-175-Flaschen aufgeteilt und zum Wachstum bei 37°C wie beschrieben inkubiert. Nach 3 Tagen und Erreichen der Konfluenz wurden die Zellen erneut nach Standardverfahren durch Trypsinbehandlung aufgeteilt auf 20 T-Flaschen. Nach weiteren 3 Tagen konnten die Zellen auf 40 T-Flaschen aufgeteilt werden. Nach weiteren 3 Tagen wurden 39 dieser T-Flaschen auf 78 T-Flaschen aufgeteilt. Da diese 78 T-Flaschen zur Ernte der Zellen verwendet werden, werden hierfür geeignete Zellkulturflaschen (Fa. Costar; Accell; Nr. 3155) benutzt. Am Tag nach dem Beimpfen dieser T-Flaschen wurden 68 Flaschen mit Phosphoramidon (Fa. Peptide Institute; Nr. 4082; abgekürzt als PHAM) in einer Endkonzentration von $10^{-4}$M versetzt (+ PHAM). Diese Behandlung führt zur Induktion der ECE-Aktivität. Die restlichen 10 T-Flaschen wurden als nicht induzierte Kontrollzellen mitgeführt (-PHAM). Nach weiteren 2 Tagen Inkubation unter den beschriebenen Bedingungen wurden die PHAM-induzierten Zellen und die Kontrollzellen (-PHAM) getrennt geerntet. Hierzu wurden die Flaschen geöffnet und das Medium abgekippt. Die Zellen wurden in der T-Flasche mit je 10 ml PBS (Boehringer Mannheim Nr. 210331) gewaschen (d.h. über Zellrasen vorsichtig geschwenkt). Das PBS wurde abgekippt. Erneut wurden 5 ml PBS pro T-Flasche gegeben. Die Zellen wurden mit einem Cell-Scraper (Fa. Costar Nr. 3010) vom Boden der Kulturflasche gekratzt und als Zellsuspension in ein 150 ml Zentrifugenröhrchen (Falcon Nr. 2076) überführt, wo sie auf Eis aufbewahrt wurden. Jede T-Flasche wurde mit 10 ml PBS nachgespült. Die Zellsuspension aus dem Nachspülen wurde mit der vorhandenen Zellsuspension in den Zentrifugenröhrchen vereinigt. Die Zellen wurden durch Zentrifugation (1200 RPM; 10 Minuten; Heraeus Christ Minifuge GL Nr. 4400; Rotor 2150) sedimentiert. Der zellfreie Überstand wurde verworfen und das Pellet im 3-fachen Volumen PBS des Zellsedimentes aufgenommen. Nach erneutem Zentrifugieren wurde der zellfreie Überstand erneut verworfen. Das Pellet blieb bis zur Aufarbeitung auf Eis.

Beispiel 2

Vergleichende Anreicherung von nicht stimulierten und mit Phosphoramiden stimulierten Rinderendothelzellen, FBHE; Identifizierung des Proteins.

Die nach Beispiel 1 erhaltenen FBHE-Zellen mit und ohne Phosphoramidon-Induktion (jeweils 500 µl Feuchtmasse) wurden in 15 ml PBS-Puffer, 0,5 mM Diisopropylfluorophosphat 20 Minuten im Eisbad mit Ultraschall behandelt. Nach Zentrifugation (20 Min. 1000 g) werden die Membranen durch Zugabe von 5 % Pluriol F68® zum Überstand gefällt und durch erneute Zentrifugation bei 10 000 g gewonnen. Die Membranen werden mit 2 ml 100 mM Tris-Puffer, pH 8,0 digeriert und mit 1 % Triton X-100® solubilisiert. Die Solubilisate (jeweils 2,5 ml) wurden zentrifugiert, die Rückstände verworfen.

Mono-Q®-Chromatographie

Die Solubilisate der +/- -Phosphoramidon-induzierten Zellen wurden getrennt auf eine Mono Q® HR 5/5-Säule (Fa. Pharmacia) unter exakt gleichen Bedingungen chromatographiert. Die Säule wird mit 50 ml Tris-Puffer, pH 8,0, 0,1 % Triton X-100 (A-Puffer) äquilibriert. Nach Auftrag des Solubilisates wird 15 Min. mit A-Puffer gewaschen und ein 50 Min-Gradient linear mit B-Puffer (A-Puffer + 1 M NaCl gefahren (Fluß 0,2 ml/min). 20 Fraktionen a 0,5 ml werden gesammelt und mit Human Big Et-1 als Substrat getestet. Das gebildete Et-1 wird mittels Reversed-Phase-HPLC in beschriebener Weise (Beispiel 3) nachgewiesen und quantifiziert. Die beiden Fraktionen mit der höchsten Enzymaktivität werden jeweils vereinigt.

Superose 12®-Gelchromatographie

Die Eluate aus den Mono Q-Chromatographien werden mittels Centricon®-Röhrchen (Fa. Amicon) auf 250 µl konzentriert und jeweils über eine Superose 12 HR10/36 gelchromatographiert.

| | | |
|---|---|---|
| Bedingungen: | Puffer: | 20 mM Natriumphosphat, 250 mM NaCl, pH 7,4, 0,05 % Triton X 100 |
| | Fluß: | 0,5 ml/min |
| | Fraktion: | 0,5 ml |

Nach dem in Beispiel 3 beschriebenen Test werden die Fraktionen getestet und die jeweils 2 Fraktionen mit der höchsten Enzymaktivität vereinigt.

Proteinbestimmungen aller Fraktionen erfolgten nach der Methode von Bradford (Anal. Biochem. 72, 248 (1976)).

Ergebnisse:

Die Reinigung von stimulierten und unstimulierten Zellen ergab folgende Ergebnisse auf den verschiedenen Stufen der Reinigung:

| Reinigungsschritt | Spez. Akt. [µU/mg] FBHE-Zellen + Phosphoramidon | µg/mg FBHE-Zellen - Phosphoramidon |
|---|---|---|
| Membranen | 129 | 44 |
| Solubilisat | 188 | 22 |
| Mono Q-Chromat. | 1470 | 78 |
| Superose 12-Chromat. | 4500 | 130 |

Die beiden aktiven Fraktionen der Superose 12®-Chromatographie wurden vergleichend auf ein 8 %iges SDS-Polyacrylamidgel geladen. Das Gelmuster von gereinigtem ECE-1 aus Phosphoramidon behandelten Zellen zeigt im Vergleich zu den nicht behandelten Zellen eine zusätzliche Bande bei 250 000 Dalton. Trägt man die Proteine mit dem Reduktionsmittel Dithiothreitol (DTT) auf, so zeigt sich im Vergleich eine zusätzliche Bande bei 125 000 Dalton.

Beispiel 3

ECE-1-Aktivitätstest mittels HPLC

Umsatz von Big-Endothelin-1 zu Endothelin-1 mittels des Endothelin-Konversionsenzyms (ECE-1)

1 µl Enzymlösung, erhalten nach Beispiel 4, wurde mit 21 µl 50 mM Tris, 50 mM Imidazol, 250 mM NaCl, pH 7,4 Puffer und 2,5 µl humanem Big-Endothelin (2 mg/ml in 0,1 % Essigsäure in Wasser) versetzt. Nach 2 Stunden wurde die Reaktion zwecks Analyse des gebildeten Endothelins mit 72 µl 0,5 % Trifluoressigsäure abgestoppt. Die Probe wurde zentrifugiert und der Überstand durch Analyse mittels Reversed Phase-Hochdruckflüssigkeitschromatographie (RP-HPLC), wie in (J. Takada et al., Biochem. Biophys. Res. Comm. 176, 860 (1991), K. Ohnaka et al., Biochem. Biophys. Res. Comm. 168, 1128 (1990)) beschrieben, identifiziert und durch Vergleich mit einem Endothelin-Standard mittels UV-Detektion (205 nm) quantifiziert.

Die Enzymaktivität läßt sich dann berechnen in Aktivität

$$ECE[\mu U] = \frac{10^{-6}\ \mu M\ \text{Endothelin}}{\text{min}}$$

Nach diesem Verfahren wurde die ECE-Aktivität der Aufarbeitung von FBHE-Zellen zu verschiedenen Stufen der Reinigung bestimmt. Gleichzeitig wurden die Proteinmengen nach der Methode von Bradford (Anal. Biochem. 72, 248 (1976)) bestimmt.

Beispiel 4

Reinigung von ECE-1 aus Rinderendothelzellen, FBHE

a) Membrangewinnung, Trypsinisierung von Fremdprotein

6 ml FBHE-Zellpellet (nach Beispiel 1) werden in 25 ml PBS-Puffer digeriert und 15 Minuten im Ultraschallbad unter Eiskühlung aufgeschlossen. Die Zelltrümmer wurden durch Abzentrifugation bei 10 000 g, 20 Min., entfernt. Der Überstand wurde unter Rühren mit 3,6 mg Trypsin versetzt und 1,5 h bei Raumtemperatur gerührt. Die Probe wurde 1 h bei 100 000 g zentrifugiert und die im Rückstand befindlichen Membranen mit 25 ml 100 mM Tris-Puffer, pH 8,0 suspendiert.

b) Solubilisierung von ECE-1

Die 25 ml Membransuspension wurden auf 0,5 mM Diisopropylfluorphosphat und anschließend 0,5 % Triton X-100® eingestellt und über Nacht im Eisbad aufbewahrt.

c) Mono-Q®-Chromatographie

Eine Mono Q®-FPLC-Säule, HR16/10, Fa. Pharmacia wurde mit 50 mM Tris-Puffer, pH 8,0; 0,05 % Triton X 100® (A-Puffer)äquilibriert.

Nach Auftrag des Solubilisates wurde 30 Min. mit A-Puffer gewaschen und danach mit einem linearen Gradienten innerhalb 100 Minuten nach Puffer B (Puffer A + 1 m NaCl) eluiert. Es werden 30 Fraktionen je 10 ml gesammelt. Der Proteingehalt der Proben wird nach der Bradford-Methode, die ECE-1 Aktivität nach Beispiel 3 bestimmt.

Die beiden Fraktionen mit der höchsten spezifischen Aktivität wurden vereinigt.

d) Gelfiltration

Die vereinigten Mono Q®-Eluate nach 4c) wurden über eine 30KDa-Centricon-Membran(Fa. Amicon) auf 500 µl konzentriert.

Eine Superose 12®, HR 10/30-Säule (Fa. Pharmacia) wird mit 20 mM Na-Phosphat-Puffer, pH 7,4, 250 mM NaCl, 0,05 % Triton X 100 äquilibriert und die konzentrierte Probe aufgetragen. Bei einem Fluß von 0,5 ml wird mit dem Äquilibrierungspuffer chromatographiert und 0,5 ml-Fraktionen gesammelt.

Die Bestimmung der spezifischen Aktivität der Fraktionen erfolgt wie in 4c). Die Fraktion mit der höchsten spezifischen Aktivität ist unter e) angegeben; sie eluiert bei ca. 250 000 Dalton, geeicht an Standardproteinen unter gleichen Chromatographiebedingungen.

e) Ergebnisse

| Reinigungsschritt | Protein [mg] | Spez. Aktivität [µU/mg] |
|---|---|---|
| Membranen | 87 | 410 |
| Solubilisat | 87 | 360 |

Tabelle fortgesetzt

| Reinigungsschritt | Protein [mg] | Spez. Aktivität [μU/mg] |
|---|---|---|
| Mono Q-Eluat | 6,6 | 3760 |
| Superose 12-Eluat | 0,35 | 15100 |

Beispiel 5

SDS-Gel-Analyse von gereinigtem ECE-1 aus FBHE-Zellen

Das nach Beispiel 4 gereinigte ECE-1 wird auf einem 8 %igen SDS-Gel nach Lämmli (Nature 227, 680 (1979)) unter nicht reduzierenden und reduzierenden Bedingungen (+DTT) analysiert. ECE-1 hat danach im nicht reduzierten Zustand ein Molekulargewicht von 250 000 Dalton und zerfällt nach Reduktion in eine breite Bande um 125 000 Dalton.

Beispiel 6

Deglykosylierung von ECE-1

50 μl der nach Beispiel 4 gereinigten ECE-1-Lösung wurden mit 10 %iger SDS-Lösung auf 0,25 %-SDS-Gehalt eingestellt. Die Proben wurden 20 Min. bei Raumtemperatur inkubiert und auf 1 % Triton X-100 eingestellt. Nach 20 Min. bei RT wurden 5 μl 250 mM Na-Phosphatpuffer, pH 4,7 zugegeben. Danach wurden 0,25 μl PNGase und 0,5 μg Endo F zugegeben. Die Proben wurden 8 h bei 37°C inkubiert. Anschließend wurden die gleichen Enzymmengen erneut zugegeben und 8 weitere Stunden bei 37°C inkubiert. Danach wurden die Proben auf 50 μl einkonzentriert und im 4 - 12 %igen SDS Gel analysiert.

Ergebnisse:

Im reduzierten Zustand verändert sich das apparente Molekulargewicht von 125 000 Dalton durch Abspaltung der Zuckerreste mit der Mischung von PNGase F/Endo F auf ca. 82 000 Dalton.

Beispiel 7

Partialsequenzen des ECE-1 aus Rinderendothelzellen

16 x 25 μg der nach Beispiel 4 erhaltenen ECE-1-Lösung wurden in 4-12 %igen SDS-Polyacrylamid-Gradientengelen präparativ aufgetragen. Nach üblicher Färbung mit Coomassie blue wird das Gel entfärbt und 4 x 40 Minuten in reinem Wasser gewässert. Die bei 250 KDa gefärbten Banden wurden mit einem Skalpell ausgeschnitten und mit 200 μl 100 mM $NH_4HCO_3$-Lösung digeriert. Nach Zugabe von 0,5 μg Trypsin wird über Nacht inkubiert, der Überstand danach abgenommen. Der Rückstand wird erneut mit 0,5 μg Trypsin 5 h bei Raumtemperatur in 200 μl 100 mM $NH_4HCO_3$-Puffer inkubiert. Danach wird die Probe im speed-vac-Konzentrator zur Trockene eingeengt. Der Rückstand wird mit 40 μl Wasser aufgenommen und mit 4 μl 40 mM Dithiothreitol-Lösung 30 Min. gerührt. Danach wurden bei 37°C 4 μl 100 mM Jodacetamid-Lösung zugegeben. Nach 2 Stunden wird die Probe zur Trockne eingeengt.

Die entstandenen Peptide wurden über eine 1 mm x 15 cm Reversed-Phase-HPLC-Säule in einem linearen 3-Stunden-Gradienten von 0,5 % Trifluoressigsäure in Wasser nach 0,5 % Trifluoressigsäure in 90 % Acetonitril aufgetrennt. UV-aktive Fraktionen wurden gesammelt und die Primärsequenzen auf einem Gasphasen-Sequenzer bestimmt.

Ergebnisse:

Folgende Partialsequenzen wurden bestimmt:

SEQ ID NO: 1:

Xaa-Xaa-Pro-Asn-Ala-Leu-Asn-Phe-Gly-Gly-Ile-Gly-Val-Val-Val-Gly-His-Glu-Leu-Thr-His-Ala-Phe...

SEQ ID NO: 2:
Xaa-Tyr-Xaa-Lys-Xaa-Gly-Asn-Leu-Arg-Pro

SEQ ID NO: 3:

Xaa-Ile-Ala-Xaa-Glu-Thr-Glu—Leu-Glu-Ile...
(Ile) (Ile)

SEQ ID NO: 4:

Xaa-Pro-Glu-Phe-Leu-Leu-Glu-Gly-Leu-Ile-Thr-Asp-Pro...

SEQ ID NO: 5:
Xaa-(Gln)-(Ala)-(Glu)-Asn-Val-Ile-Gln-Val-Xaa-Gln...

SEQ ID NO: 6:
Val-Glu-Ile-Val-Phe-Pro-Ala-Gly-Ile-Leu-Gln-Ala-Pro-(Phe)-Tyr-Thr
(Thr)

Die in Klammern angegebenen Aminosäuren wurden nicht absolut sicher identifiziert.
SEQ ID NO: 1 belegt, daß es sich beim ECE-1 um ein neues Protein aus der Familie der Metalloproteasen handelt, da es im Vergleich mit Sequenzen der Metallendopeptidasen NEP 24.11 und Thermolysin signifikante Homologien von 72 % bzw. 40 % aufweist.

Beispiel 8

Herstellung einer cDNA-Sequenz, die für ein Endothelinkonversionsenzym kodiert

a) Isolierung von RNA und Herstellung einer cDNA-Bank

Gesamt-RNA aus FBHE-Zellen, die nach Beispiel 1 mit Phosphoramidon stimuliert wurden, oder aus HeLa-Zellen, die nach Beispiel 1 mit Phosphoramidon stimuliert wurden, wurde durch Aufschluß in Guanidiniumthiocyanat gewonnen. Dabei wurde mit Materialien und nach Anleitung des "RNA Isolation Kit" der Firma Stratagene, La Jolla, CA, USA (Catalog Nr.200345) gearbeitet.
Die polyadenylierte messenger RNA wurde aus der o.a. Gesamt-RNA aus FBHE-Zellen durch oligo(dT)-Affinitätsseparation selektiert. Dieses Verfahren wurde mit Materialien und nach Anleitung des "PolyATtract mRNA Isolation System" der Firma Promega, Madison, WI, USA (Catalog Nr.Z5200) durchgeführt. Aus polyadenylierter messenger RNA wurde mit Materialien und nach Anleitung des "ZAP-cDNA Synthesis Kit' der Firma Stratagene, La Jolla, CA, USA (Catalog Nr.200400) cDNA synthetisiert, die dann mit Materialien und nach Anleitung des "Uni-ZAP XR GigapackII Cloning Kit' der Firma Stratagene, La Jolla, CA, USA (Catalog Nr.237611) in Lambda Phagen verpackt wurde.

b) Herstellung von Oligonukleotidproben für die "RACE"-PCR

Zur Klonierung von cDNA-Fragmenten mit Hilfe der Polymerase-Kettenreaktion (PCR, s. 'Molecular Cloning', 2nd edition (1989), Sambrook, J. et al., CSH-Press, Seite 14.1 ff.) wurde von den in Beispiel 7 angeführten Peptiden mit den SEQ ID NO: 1 und NO: 6 ausgegangen.

Unter Zugrundelegung des genetischen Codes läßt sich aus er SEQ ID NO: 1, Position 16 bis 22 ein Oligonukleotid-Gemisch mit der Nukleinsäuresequenz SEQ ID NO: 7 herleiten:

5'-GGSCAYGARYTNACNCAYGC-3'.

Desgleichen lassen sich aus der SEQ ID NO: 6, Position 2 bis 8 ein Oligonukleotidgemisch mit der SEQ ID NO: 8:

5'-GARATYGTSTTYCCYGCYGG-3',

sowie aus der SEQ ID NO: 6, Position 9 bis 16 ein Oligonukleotidgemisch mit der SEQ ID NO: 9:

5'-ATYCTSCAGGCYCCYTTYTAYAC-3'

herleiten.

Dabei entsprechen SEQ ID NO: 7 bis NO: 9 der Sequenz des kodierenden DNA-Stranges. Wegen der bekannten Degeneriertheit des genetischen Codes sind an manchen Positionen mehrere Nukleotide eingesetzt worden. Damit ergibt sich für SEQ ID NO: 7 bis 9 eine bis zu 512fache Gemischkomplexität. Die genannten Sequenzen wurden als Oligonukleotide synthetisiert.

Folgende Oligonukleotide wurden zusätzlich als 3'Primer in der "RACE"-PCR synthetisiert:

A-B-T18 (SEQ ID NO: 10):
5'-CGAGGGGGATGGTCGACGGAAGCGACCTTTTTTTTTTTTTTTTTT-3';

sowie

A (aus A-B-T18) (SEQ ID NO: 11):
5'-CGAGGGGGATGGTCGACGG-3';

sowie

B (aus A-B-T18) (SEQ ID NO: 12):
5'-GATGGTCGACGGAAGCGACC-3'.

Die Synthesen wurden mit einem Applied Biosystems Typ 360A DNA-Synthesizer durchgeführt. Die Oligonukleotide wurden nach Entfernung der Schutzgruppen gelelektrophoretisch über ein Acrylamid/Harnstoff-Gel gereinigt.

c) Herstellung von DNA-templates für die PCR

5 μg Gesamt-RNA oder 1 μg Poly(A)+-RNA der unter a) aufgeführten RNA-Präparation aus Phosphoramidonstimulierten FBHE Zellen wurden mit 1 μg des Oligonukleotides A-B-T18 (SEQ ID NO: 10) und mit Hilfe des Enzyms Reverse Transkriptase in einzelsträngige cDNA (1°cDNA) übersetzt. Dabei wurde mit Materialien und nach Anleitung des "SuperScript Preamplification System" der Firma Gibco BRL, Eggenstein, Deutschland (Catalog Nr.8089SA) gearbeitet. Nach Beendigung der Reaktion wurden die Syntheseprodukte mittels "Geneclean II Kit" der Firma BIO 101, La Jolla, CA, USA, von kleineren Molekülen und überschüssigen Oligonukleotiden gereinigt.

d) PCRs und Klonierung einer Teil-cDNA-Sequenz

Die Polymerase-Kettenreaktion wurde nach bekannten Protokollen durchgeführt (s. "Molecular Cloning", 2nd edition (1989), Sambrook, J. et al., CSH-Press, Seite 14.1 ff.). Dazu wurde ein "DNA Thermal Cycler" der Firma Perkin Elmer benutzt. Dabei wurde das Prinzip der "verschachtelten Primer" nach Frohmann, M.A. et al. (Proc.Natl.Acad.Sci.USA (1988) 85, 8998-9002) verwendet und nach Fritz, J.D. et al. (Nucl.Acids Res. (1991) 19, 3747) modifiziert angewendet.

Im Einzelnen wurde die 1°cDNA aus c) mit jeweils 20 pmol der Oligonukleotide SEQ ID NO: 8 und A (aus A-B-T18) amplifiziert. Die Bedingungen waren dabei: 1' 95°C; 2' 55°C; 3' 72°C für 35 Zyklen.
Die PCR-Produkte wurden elektrophoretisch auf einem 1,2 %igen LMP-Agarose/TBE-Gel aufgetrennt.
Aus dem Gel wurden über die gesamte Länge des "Schmiers" etwa 10 Gelscheibchen geschnitten und diese als separate Fraktionen mit DNA-Fragmenten steigender Molmasse geschmolzen.

Aliquots dieser Fraktionen wurden dann separat in eine zweite PCR mit jeweils 20 pmol der Oligonukleotide SEQ ID NO: 9 und B (aus A-B-T18) eingesetzt. Dabei überschritt der Agaroseanteil nie 1/10 Volumen des PCR-Ansatzes. Reaktionsbedingungen: 1'95°C; 2'50°C; 3'72°C für 35 Zyklen.

Die gelelektrophoretische Auftrennung der Amplifikationsprodukte dieser Fraktionen ließ deutlich eine Reduzierung des komplexen Produktspektrums der ersten PCR hin zu einer definierten Bande von ca. 1000 bp Länge nach der zweiten PCR erkennen.

Dieses solchermaßen selektierte PCR-Produkt wurde nach Standardmethoden eluiert. Die Identität der 1000 bp großen Bande als ein Fragment der bovinen Endothelinkonversionsenzym cDNA wurde zunächst durch eine weitere, dritte PCR-Amplifikation mit den Oligonukleotiden SEQ ID NO: 7 und B (aus A-B-T18) überprüft. Das Produkt dieser PCR war eine ca. 950 bp große Bande.

Nach Subklonierung der 1000 bp großen Bande aus der zweiten PCR-Reaktion in den Vektor pCR II (TA Cloning Kit, Invitrogen Corp., San Diego Cat.No. K2000-01) und Vermehrung des Plasmides in E.coli DH5alpha ergab die Sequenzanalyse eines Klones ein offenes Leseraster von 189 Aminosäuren SEQ ID NO: 13, NO: 14). Im gleichen Leseraster fanden sich auch die Sequenzen der Peptide SEQ ID NO: 1, NO: 2 und NO: 4, wodurch die Identität des cDNA Fragmentes eindeutig definiert wird.

e) Klonierung einer bovinen cDNA für das Endothelinkonversionsenzym

Nach dem in Beispiel 1a) aufgeführten Protokoll wurde eine cDNA-Bibliothek mit einem statistischen Primergemisch im Schritt der Erststrang-Synthese generiert. Die Primer wurden als Sequenz:

5'-GAGAGAGAGTCGACGGTACCN7 ; SEQ ID NO: 15

synthetisiert.

Abweichend von o.a. cDNA-Syntheseprotokoll wurde die doppelsträngige cDNA über eine 2 ml Säule S-1000 Sephacryl (Grad: "Superfine"; Pharmacia, Freiburg; Catalog Nr. 17-0476-01) fraktioniert. Die Fraktionen mit cDNA-Fragmenten von mindestens 1 kb Größe wurden durch Alkoholfällung auf bekannte Weise konzentriert und entsprechend der Anweisung im cDNA-Synthese Kit weiterbehandelt, wobei die cDNA-Präparation mit der Restriktionsendonuklease SalI statt mit XhoI nachgespalten und in den Lambdavektor integriert wurde. 2 x 106 Klone der Bank wurden mit einer DNA-Sonde, die mit den Oligonukleotiden

5'-CGGCCCTGGTGGAAGAACTCG-3' (SEQ ID NO: 16)

und

5'-TGCGGACGGAACACCAGACCT-3' (SEQ ID NO: 17)

aus der partiellen Rinder cDNA (SEQ ID NO:13), Position 136 bis 156, bzw. 391 bis 412 erzeugt wurde, nach Transfer auf Nylonmembranen hybridisiert. Die DNA-Sonde wurde in einer Polymerase-Kettenreaktion (PCR) in Gegenwart von DigoxigenindUTP markiert, wie in Beispiel 1f) beschrieben. Die Hybridisierung wurde unter stringenten Bedingungen durchgeführt (s. Beispiel 1f)), wobei der letzte Waschschritt nach erfolgter Hybridisation in 0.1 x SSC, 60°C durchgeführt wurde und anschließend der immunologische Nachweis der gebundenen DNA-Sonde erfolgte. Die Sequenzierung ausgewählter Klone ergab die Rinder cDNA-Sequenz C60, SEQ ID NO: 18, mit einem durchgehenden Leserahmen, SEQ ID NO: 19.

Danach wurden 2 x 106 Klone der Bank mit einer DNA-Sonde, die mit den Oligonukleotiden

5'-GCCAGCGCCGTCTCAAAGTCCAG-3'. (SEQ ID NO: 20)

und

5'-TGGGGGACCTTCAGCAACCTCT-3' (SEQ ID NO: 21)

aus dem cDNA-Klon C60, SEQ ID NO: 18, Position 500 bis 522, bzw. 14 bis 35, wie oben beschrieben, erzeugt wurde, unter gleichen Bedingungen hybridisiert. Die Sequenzierung ausgewählter Klone ergab die Rinder cDNA-Sequenz SEQ ID NO: 22 mit einem durchgehenden Leserahmen von 708 Aminosäuren (SEQ ID NO: 23).

5 x 108 plaquebildende Einheiten einer käuflichen Rinderlungen cDNA Bank in Lambda gt11 (Clontech Laboratories, Inc.; 4030 Fabian Way; Palo Alto CA 94303-4607, USA; Katalog Nr. BL1006b) wurden durch Fällung mit 10 % Polyethylenglykol 6000, 1 M NaCl konzentriert in 100 µl Bidest resuspendiert und 5 min auf 70°C erhitzt. 5 µl dieses Lysats wurden in einer 50 µl Standard PCR-Reaktion (Beispiel 8d) mit den Primern gt11 fwd und 5'-GGTGCTTGATGATGGCTTGGTTGT-3' (SEQ ID NO: 26) eingesetzt. Der Primer gt11 fwd entspricht der Position 2979-3003 des β-Galaktosidasegens (Genbank: ECLACZ) und liegt im Klonierungsvektor Lambda gt11 unmittelbar vor der singulären EcoRI-Integrationsstelle, die bei der Bankkonstruktion benutzt wurde (Young, R.A. u. Davis, R.W. (1985) Genetic Engineering, Ed. by Setlow, J. u. Hollander, A. Plenum Press, N.Y. 29-41). Der Primer SEQ ID NO: 26 entspricht der Position 280-303 der SEQ ID NO: 22.

Die Amplifikationen wurden über 40 Zyklen durchgeführt. Das PCR-Produkt wurde auf einem 3 % "Low Melting Point' Nu-Sieve GTG-Agarosegel (Firma FMC BioProducts, 191 Thomaston Street, Rockland, ME 04841, USA; Katalog Nr. 50082) aufgetrennt und der Größenbereich 400-600 Basenpaare durch Ausschneiden einzelner Gelsegmente fraktioniert. Die Gelscheiben wurden, wie im Beispiel 8d) beschrieben, aufgeschmolzen, und 1.5 µl wurden in 50 µl einer frischen PCR-Reaktion mit den Primern gt11 fwd und 5'-AGATGGAGCTGGTCACCGAGATGC-3' (SEQ ID NO: 27) eingesetzt. Der Primer SEQ ID NO: 27 entspricht der Position 127-150 der SEQ ID NO: 22.

Die entstandenen PCR-Fragmente wurden nach Subklonierung in dem Plasmidvektor pUC18 (Yanisch-Perron, D., Vieira, J. u. Messing, J. (1985) Gene 33, 103-119) sequenziert (SEQ ID NO: 28).

Diese Sequenz überlappt mit den Nukleotiden 175-324 die SEQ ID NO: 22 in den Positionen 1-150 und verlängert sie um 174 Nukleotide in 5'-Richtung. Die resultierende Gesamtsequenz (SEQ ID NO: 29) kodiert einen offenen Leserahmen von 754 Aminosäuren (SEQ ID NO: 30).

f) Klonierung einer humanen cDNA für das Endothelinkonversionsenzym

Da Endothelin-1 in humaner Plazenta nachgewiesen worden war, wurde angenommen, daß das Endothelinkonversionsenzym auch in Plazenta exprimiert wird. Es wurde daher unter Verwendung einer markierten Rinder-cDNA-Sonde eine humane λgt-11 Plazenta cDNA Bank gescreent. Zur Herstellung der Digoxigenin-dUTP markierten Rinder-cDNA-Sonde wurde in einer PCR-Reaktion (siehe "Molecular Cloning", 2nd edition (1989), Sambrook, J. et al., CSH-Press, Seite 14.1 ff.) als 'template' 1 ng (1ng/ml) der partiellen Rinder-cDNA-Sequenz (SEQ ID NO: 13) verwendet. Das 'sense'-Oligonukleotid (SEQ ID NO: 16) umfaßte die Nukleotide von Position 136 bis 156 in SEQ ID NO: 13, das 'antisense'-Oligonukleotid SEQ ID NO: 17) die Positionen 392 bis 412 in der SEQ ID NO: 13. Im 'DNA Thermal Cycler' (Perkin Elmer) wurden die nachfolgenden Zyklen 35 mal durchlaufen: 2 min. 94°C, 2 min 60°C und 3 min 72°C. Die markierte 276 bp Rinder cDNA-Sonde wurde anschließend über ein Agarosegel gereinigt und durch Aufkochen denaturiert. Unter niederstringenten Bedingungen wurden ca. 650.000 Klone einer humanen Plazenta λgt-11 cDNA-Bank (Clontech, HL 1008b) mit dieser Sonde gescreent. Die Hybridisierung der Filterabzüge mit der Sonde erfolgte über Nacht in 5 x SSC, 2 % Blockierreagenz (Boehringer Mannheim; No. 1096176), 0,1 % N-Laurylsarkosin, 0,02 % SDS bei 60°C. Die Filter wurden dann 2 x 5 min bei 60°C mit 2 x SSC, 0,1 % SDS und anschließend 20 min bei 60°C mit 0,5 x SSC, 0,1 % SDS gewaschen. Die weitere Entwicklung der Filter zur Identifizierung der gebundenen DIG-Sonden erfolgte entsprechend dem Protokoll von Boehringer Mannheim (DIG - Nukleinsäurendedektionskit, No. 1175041).

Es wurden Klone mit der in SEQ ID NO: 24 angegebenen cDNA-Sequenz isoliert. Die Nukleotide in der Position 1 bis Position 2109 der cDNA-Sequenz mit der SEQ ID NO: 24 kodieren für die in SEQ ID NO: 25 angegebene Aminosäuresequenz. Die Aminosäuresequenz entspricht dem größten Teil der Primärsequenz des Endothelinkonversionsenzyms, da sich die Peptide SEQ ID NO: 1 bis SEQ ID NO: 6 aus der Trypsinpeptidsequenzierung des isolierten Endothelinkonversionsenzyms in der Sequenz wiederfinden lassen. Auch die Metalloproteinasekonsensussequenz HEXXH kann als HELTH an den Positionen 540 bis 544 in der Aminosäuresequenz SEQ ID NO: 25 identifiziert werden.

Von 3 µg Plazenta poly A(+) RNA wurden wie im Beispiel 8 e) beschrieben mit dem Oligonukleotid 5'-GAGAGAGAGAGAGAGAGAGAACTAGTCTCGAGCCAAGCAGGCCACCAGTCCTG-3' (SEQ ID NO: 31) als Erst-Strang cDNA Syntheseprimer eine cDNA-Bibliothek erzeugt. Die Nukleotide 32 bis 53 entsprechen den Positionen 32 bis 53 der SEQ ID NO: 24. Die cDNA-Präparation wurde wie in Beispiel 8a) beschrieben in den Lambdavektor Uni-ZAP XR integriert. Die resultierenden 4 x105 unabhängigen Klone wurden amplifiziert und 5x108 plaquebildende Einheiten wurden, wie in Beispiel 8 e) beschrieben, in eine 100 µl PCR-Reaktion eingesetzt. Dazu wurden die Primer SEQ ID NO: 31 und C eingesetzt. Der Primer C liegt im Lambdavektor in dem Bluescript SK(-)-Teil, Pos. 881-904, Sequenzfile Genbank: ARBLSKM.

Die PCR-Reaktion wurde 40 Zyklen bei einer Hybridisationstemperatur von 65°C durchgeführt. 1 µl des Reaktionsprodukts wurde in einen frischen 50 µl PCR-Reaktionsansatz gegeben, der 40 Zyklen bei einer Hybridisationstemperatur von 60°C durchgeführt wurde. Als Primer wurden die Oligonukleotide D und

5'-CCTGCCGCCAGAAGTACCACCAACA-3' (SEQ ID NO: 32)

verwendet.

Primer D liegt im Bluescript SK(-)-Teil des Lambda Uni-ZAP XR-Vektors (Pos. 857-880, Sequenzfile Genbank:ARBLSKM), der Primer SEQ ID NO: 32 entspricht der Position 11-35 in der SEQ ID NO: 24. Das Reaktionsprodukt wurde in dem Plasmidvektor pUC18 wie oben beschrieben subkloniert. Ausgewählte Klone wurden sequenziert.

Als Sequenz wurde ein neuer 5'-Anteil der humanen ECE cDNA erhalten (SEQ ID NO: 33, dessen 3'-terminaler Bereich (Pos. 188-222) mit der Position 1-35 der SEQ ID NO: 24 verlappt. Er verlängert sie um 187 Nukleotide in 5'-Richtung und ergibt die gesamte humane ECE-Sequenz SEQ ID NO: 35, die einen offenen Leserahmen von 753 Aminosäuren (SEQ ID NO: 36) kodiert.

Beispiel 9

Herstellung von rekombinantem ECE in Säugerzellen

1. Konstruktion eines Expressionsvektors für membranstandiges humanes ECE

Zur Expression des vollständigen humanen ECE wurde die cDNA-Sequenz (SEQ ID NO: 35) von Nukleotid 29 bis 2720 mit geeigneten Adaptoren in den Expressionsvektor pcDNA3neo der Fa. Invitrogen (3985 B Sorrento Valley Blvd., San Diego, CA 92121, USA; Produkt Nr. V790-20) zwischen die Kpn I-und Xba I-Restriktionsstellen eingefügt. Die ECE-Sequenz bringt ihre eigenen Translationsstart und -stopsequenzen sowie eine Konsensus-Kozaksequenz mit (Kozak, M. (1989) J. Cell Biol. 108, 229-241). Die Transkription der ECE-Messenger RNA steht in diesem Vektor unter der Kontrolle des starken Cytomegalievieruspromotors. Die Selektion transfizierter Zellen wird über das im Plasmid liegende Neomycin-Resistenzgen durchgeführt, das nur G418-resistenten Kolonien das Wachstum erlaubt.

2. Konstruktion eines Expressionssystems für sezerniertes humanes ECE

a) Die Klonierung wurde in dem kommerziell erhältlichen eukaryontischen Expressionsvektor pcDNA3neo der Fa. Invitrogen (3985 B Sorrento Valley Blvd., San Diego, CA 92121, USA; Produkt Nr. V790-20) durchgeführt. Dazu wurde der Vektor zunächst mit den Restriktionsenzymen Eco RI und Eco RV gespalten.
b) Dann wurde aus der cDNA des humanen ECE (SEQ ID NO: 35) das Nukleinsäurefragment Position 241 - Position 2396 durch eine Polymerase-Kettenreaktion mit geeigneten Oligonukleotidprimern gewonnen. Bedingt durch die Wahl der Sequenz des 5' Primers wird die Nukleinsäuresequenz zwischen Position 241 und Position 245 (SEQ ID NO: 35) in die Basenabfolge 5'- ACGCG -3' abgeändert. Unter Einbeziehung der Position 246 wurde durch diesen Schritt eine Erkennungssequenz für die Restriktionsendonuklease Mlu I generiert. Das entstandene ECE-Fragment wurde in einem weiteren Schritt mit Mlu I gespalten.
c) Als weiteres Fragment wurde aus zwei synthetischen Oligonukleotiden (Strang/Gegenstrang) die kodierende Sequenz des humanen Gewebe-Plasminogen-Aktivators (t-PA) zwischen Nukleotid 74 und Nukleotid 176 der publizierten Sequenz (D. Collen, Nature 301, 214-221 (1983)) hergestellt. Zusätzlich zur angegebenen Sequenz wurden die synthetischen Oligonukleotide mit Adaptoren versehen, die zu einem 5' Eco RI und einem 3' Mlu I Überhang führen.

Die Ligation der unter b) und c) generierten Fragmente über die gemeinsame Mlu I Restriktionsschnittstelle führt zu einer Fusion der Leseraster des Signalpeptides des humanen t-PA Gens und der extrazellulären Domäne des humanen ECE Gens. Die Ligation dieses Fusionsproduktes in den Eco RI und Eco RV geschnittenen pcDNA3 Vektor aus a) führt zu einem Expressionssystem für sezerniertes humanes ECE.

3. Expression in Säugerzellen

Die DNA der Expressionsvektoren wurde mit Lipofektamin (Fa. Gibco; Life Technologies GmbH; Dieselstraße 5, 76334 Eggenstein, Deutschland; Nr. 530-8324SA) in Säugerzellen transfiziert. Die verwendeten Zellen waren CHO-K1 (ATCC CCL 61); BHK-21 (ATCC CCL 10); 293 (ATCC CRL 1573) und C127I (ATCC CRL 1616).

Je $2x10^5$ Zellen wurden in 3 ml Wachstumsmedium pro Napf einer 6-well-Kulturplatte eingesät. Am nächsten Tag wurde die Transfektion durchgeführt. Hierzu wurden die Zellen einmal mit PBS gewaschen. Die Transfektion mit Lipofektamin wurde nach Angaben der Herstellerfirma Fa. Gibco durchgeführt (Focus (1993), 15 Nr. 3, 73-78). Jeder Napf erhielt 1 μg DNA und 6 μl Lipofektamin, die in zusammen 1000 μl serumfreiem Zellkulturmedium aufgebracht wurden. Nach 6 stündiger Inkubation bei 37°C wurden die Zellen mit PBS einmal gewaschen und mit normalem Wachstumsmedium über Nacht inkubiert. Am nächsten Tag wurden die Zellen eines Napfes nach Ablösen mittels Trypsin auf 1, 5 oder 10 Petrischalen (Durchmesser 10 cm) verteilt. Am Tag darauf wurden die tranfizierten Zellen durch Behandlung mit G418 (Fa. Gibco; Kat.Nr. 066-01811Y; Warenname: Geneticin) selektiert, d.h. das Wachstumsmedium wurde gegen ein Medium, welches 1,2 mg/ml G418 enthielt, ausgetauscht. Nach 7 -10 Tagen waren in den Petrischalen Kolonien G418-resistenter Zellen zu beobachten. Diese wurden mit der Methode der "cloning-cylinder" (DNA-cloning Vol. II; ed. D. M. Glover, IRL press, 1985; S. 220) isoliert. Die isolierten Kolonien wurden in jeweils einen Napf einer 24-Napfschale mit ca. 1,5 ml wachstumsmedium (inklusive G418) gegeben. Bei Erreichen der Konfluenz wurden die Zellen durch Trypsinierung in größere Kulturgefäße umgesetzt.

a) membranständiges ECE

Zellen, die das membranständige ECE exprimieren, wurden auf vorliegen des ECE nach Zellaufschluß und Fraktionierung untersucht wie in Beispiel 1 und 2 beschrieben. (Eine Phosphoramidon-Stimulation wird nicht vorgenommen.) Die spezifische Aktivität der analysierten Kolonien betrug auf Membranbasis bis zu 1550 μU/mg Protein. Membranen der Kolonie 38 wurden weiter aufgearbeitet. Dabei wurde folgendes Ergebnis erhalten:

| Reinigungsschritt | spez. Aktivität (μU/mg Protein) |
|---|---|
| Membranen | 1550 |
| Solubilisat | 2010 |
| Mono-Q-Chromatographie | 12000 |

b) sezerniertes ECE

Zellen, die das ECE ohne Membrananker exprimieren und in das Zellkulturmedium sezernieren, wurden auf Vorliegen des rekombinanten ECE getestet, indem der Zellkulturüberstand von konfluenten Zellen auf ECE-Aktivität untersucht wurde. Hierzu wurde der Zellkulturüberstand nach 2 Tagen Kulturdauer abgenommen und durch Zentrifugation bei 1000 g von Zelltrümmern befreit. 6 ml des Überstandes wurden anschließend mittels einer Centricon 10.000 (Fa. Amicon, W.R. Grace + Co., Danver, Ma. 01923, USA; Product No. 4206) und Zentrifugation bei 3220 g (ca. 30 min.) konzentriert. Die durchgelaufene Flüssigkeit mit den niedermolekularen Substanzen wurde aufgehoben ("Centricon-Eluat"). Die Centricon wurde einmal mit 1 ml PBS + 10 mM Tris + 150 mM NaCl; pH 7,5 gewaschen. Das Konzentrat wurde in 300 μl des "Centricon-Eluates" aufgenommen.

18 μl des Konzentrates wurden in den ECE-Test eingesetzt wie in Beispiel 3 beschrieben.

Die Volumenaktivität des sezernierten ECE betrug je nach untersuchter Kolonie der rekombinanten Zellen bis zu 10 μUnits/ml Zellkulturmedium.

## Patentansprüche

1. Endothelinkonversionsenzym, **dadurch gekennzeichnet, dass** es die in SEQ ID NO: 36 beschriebene polypeptidsequenz oder Teilsequenzen, die noch die enzymatische Aktivität des Endothelinkonversionsenzyms besitzen, davon enthält.

2. DNA-Sequenz, die für das Endothelinkonversionsenzym nach Anspruch 1 kodiert und die aus der Gruppe ausgewählt ist, die

   a) DNA-Sequenzen mit der in SEQ ID NO: 35 beschriebenen Sequenz und
   b) DNA-Sequenzen, die für Proteine mit der in SEQ ID NO: 36 beschriebenen Sequenz kodieren,

   umfaßt.

3. Verfahren zur gentechnischen Herstellung von Endothelinkonversionsenzymen unter Verwendung von DNA-Sequenzen gemäß Anspruch 2.

4. Verfahren zur Herstellung von Endothelinkonversionsenzym gemäß Anspruch 1, **dadurch gekennzeichnet, dass** Säugerzellen mit einem Inhibitor des Endothelinkonversionsenzyms zur Endothelinkonversionsenzym-Überproduktion stimuliert werden und aus diesen Zellen Endothelinkonversionsenzym mit üblichen proteinchemischen Maßnahmen isoliert wird.

5. Verfahren nach Anspruch 4, **dadurch gekennzeichnet, dass** als Inhibitor Phosphoramidon verwendet wird.

6. Verfahren zur Herstellung von Endothelinkonversionsenzym gemäß Anspruch 1, **dadurch gekennzeichnet, dass** ein für eine Teilsequenz von SEQ ID NO: 36 kodierendes oder sequenzkomplementäres Oligonukleotid als Hybridisierungsprobe verwendet wird, um ein Gen für ein Endothelinkonversionsenzym aus einem Genpool zu isolieren und dieses Gen mit üblichen gentechnischen Methoden in einem nicht-menschlichen Wirtsorganismus zu exprimieren.

7. Verwendung von Endothelinkonversionsenzym gemäß Anspruch 1 zur Identifizierung von Hemmstoffen des Endothelinkonversionsenzyms.

8. Verwendung von Endothelinkonversionsenzym gemäß Anspruch 1 zur Herstellung von Antikörpern.

9. Verwendung einer DNA-Sequenz gemäß Anspruch 2 zur Herstellung von nicht-menschlichen transgenen Tieren, die ein oder mehrere Extrakopien dieser DNA-Sequenz enthalten oder bei denen das normale ECE-Gen ausgeschaltet ist.

10. Verwendung von Endothelinkonversionsenzym gemäß Anspruch 1 zur Herstellung von Arzneimitteln.

## Claims

1. Endothelin-converting enzyme, **characterised in that** it contains the polypeptide sequence described in SEQ ID NO: 36 or partial sequences thereof that still possess the enzymatic activity of the endothelin-converting enzyme.

2. DNA sequence that codes for the endothelin-converting enzyme according to claim 1 and that is selected from the group consisting of

   a) DNA sequences having the sequence described in SEQ ID NO: 35 and
   b) DNA sequences that code for proteins having the sequence described in SEQ ID NO: 36.

3. Process for the production of endothelin-converting enzymes by genetic engineering using DNA sequences according to claim 2.

4. Process for the production of endothelin-converting enzyme according to claim 1, **characterised in that** mammalian cells are stimulated to overproduce endothelin-converting enzyme by means of an inhibitor of the endothelin-converting enzyme, and endothelin-converting enzyme is isolated from those cells by conventional measures of protein chemistry.

5. Process according to claim 4, **characterised in that** phosphoramidone is used as the inhibitor.

6. Process for the production of endothelin-converting enzyme according to claim 1, **characterised in that** an oligonucleotide coding for a partial sequence of SEQ ID NO: 36 or a sequence-complementary oligonucleotide is used as hybridisation probe in order to isolate a gene for an endothelin-converting enzyme from a gene pool and express that gene in a non-human host organism by conventional methods of genetic engineering.

7. Use of endothelin-converting enzyme according to claim 1 in the identification of inhibitors of the endothelin-converting enzyme.

8. Use of endothelin-converting enzyme according to claim 1 in the production of antibodies.

9. Use of a DNA sequence according to claim 2 in the production of non-human transgenic animals that contain one or more extra copies of that DNA sequence or in which the normal ECE gene is excluded.

10. Use of endothelin-converting enzyme according to claim 1 in the production of medicaments.

**Revendications**

1. Enzyme de conversion de l'endothéline **caractérisée en ce qu'**elle contient la séquence polypeptidique décrite dans SEQ ID NO: 36 ou des séquences partielles de celle-ci qui possèdent encore l'activité enzymatique de l'enzyme de conversion de l'endothéline.

2. Séquence d'ADN qui code l'enzyme de conversion de l'endothéline selon la revendication 1 et qui est choisie dans le groupe qui comprend :

   (a) les séquences d'ADN ayant la séquence décrite dans SEQ ID NO: 35 et
   (b) les séquences d'ADN qui codent des protéines ayant la séquence décrite dans SEQ ID NO:36.

3. Procédé de production par génie génétique d'enzymes de conversion de l'endothéline au moyen de séquences d'ADN selon la revendication 2.

4. Procédé de production de l'enzyme de conversion de l'endothéline selon la revendication 1 **caractérisé en ce que** des cellules de mammifère sont stimulées avec un inhibiteur de l'enzyme de conversion de l'endothéline pour la surproduction d'enzyme de conversion de l'endothéline et l'enzyme de conversion de l'endothéline est isolée à partir de ces cellules avec des mesures de chimie des protéines courantes.

5. Procédé selon la revendication 4 **caractérisé en ce que** le phosphoramidon est utilisé comme inhibiteur.

6. Procédé de production de l'enzyme de conversion de l'endothéline selon la revendication 1 **caractérisé en ce qu'**un oligonucléotide codant une séquence partielle de SEQ ID NO: 36 ou de séquence complémentaire est utilisé comme sonde d'hybridation pour isoler à partir d'un pool de gènes un gène pour une enzyme de conversion de l'endothéline et pour exprimer ce gène dans un organisme hôte non humain avec des méthodes de génie génétique habituelles.

**7.** Utilisation de l'enzyme de conversion de l'endothéline selon la revendication 1 pour l'identification de substances inhibitrices de l'enzyme de conversion de l'endothéline.

**8.** Utilisation de l'enzyme de conversion de l'endothéline selon la revendication 1 pour la production d'anticorps.

**9.** Utilisation d'une séquence d'ADN selon la revendication 2 pour la production d'animaux transgéniques non humains qui contiennent une ou plusieurs copies supplémentaires de cette séquence d'ADN ou chez lesquels le gène de ECE normal est inactivé.

**10.** Utilisation de l'enzyme de conversion de l'endothéline selon la revendication 1 pour la production de médicaments.

SEQUENZPROTOKOLL

(1) ALLGEMEINE INFORMATION:

(i) ANMELDER:

(A) NAME: BASF Aktiengesellschaft
(B) STRASSE: Carl-Bosch-Strasse 38
(C) ORT: Ludwigshafen
(E) LAND: Bundesrepublik Deutschland
(F) POSTLEITZAHL: D-67056
(G) TELEPHON: 0621/6048526
(H) TELEFAX: 0621/6043123
(I) TELEX: 1762175170

(ii) ANMELDETITEL: Endothelinkonversionsenzym (ECE)
(iii) ANZAHL DER SEQUENZEN: 25
(iv) COMPUTER-LESBARE FORM:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:

(A) LÄNGE: 23 Aminosäuren
(B) ART: Aminosäure
(C) STRANGFORM: Einzel
(D) TOPOLOGIE: linear

(ii) ART DES MOLEKÜLS: Peptid
(iii) HYPOTHETISCH: NEIN
(v) ART DES FRAGMENTS: inneres
(vi) URSPRÜNLICHE HERKUNFT:

(A) ORGANISMUS: Bos taurus
(F) GEWEBETYP: Endothelial
(H) ZELLLINIE: Fötale Rinderherz Endothelzellinie

(xi) SEQUENZBESCHREIBUNG: SEQ ID NO: 1:

```
Xaa Xaa Pro Asn Ala Leu Asn Phe Gly Gly Ile Gly Val Val Val Gly
1               5                   10                  15

His Glu Leu Thr His Ala Phe
            20
```

(2) INFORMATION ZU SEQ ID NO: 2:

(i) SEQUENZ CHARAKTERISTIKA:

(A) LÄNGE: 10 Aminosäuren
(B) ART: Aminosäure
(C) STRANGFORM: Einzel
(D) TOPOLOGIE: linear

(ii) ART DES MOLEKÜLS: Peptid

(v) ART DES FRAGMENTS: inneres

(vi) URSPRÜNLICHE HERKUNFT:

(A) ORGANISMUS: Bos taurus

(H) ZELLLINIE: FötaleRinderherz Endothelzellinie

(xi) SEQUENZBESCHREIBUNG: SEQ ID NO: 2:

```
Xaa Tyr Xaa Lys Xaa Gly Asn Leu Arg Pro
1               5                   10
```

(2) INFORMATION ZU SEQ ID NO: 3:

(i) SEQUENZ CHARAKTERISTIKA:

(A) LÄNGE: 10 Aminosäuren

(B) ART: Aminosäure

(C) STRANGFORM: Einzel

(D) TOPOLOGIE: linear

(ii) ART DES MOLEKÜLS: Peptid

(v) ART DES FRAGMENTS: inneres

(vi) URSPRÜNLICHE HERKUNFT:

(A) ORGANISMUS: Bos taurus

(H) ZELLLINIE: Rinderherz Endothelzellinie

(ix) MERKMALE:

(A) NAME/SCHLÜSSEL: Modified-site

(B) LAGE: 8

(D) SONSTIGE ANGABEN: /note= "Xaa = Leu oder Ile"

(ix) MERKMALE:

(A) NAME/SCHLÜSSEL: Modified-site

(B) LAGE: 7

(D) SONSTIGE ANGABEN: /note= "Xaa = Glu oder Ile"

(xi) SEQUENZBESCHREIBUNG: SEQ ID NO: 3:

```
Xaa Ile Ala Xaa Glu Thr Xaa Xaa Glu Ile
1               5                   10
```

(2) INFORMATION ZU SEQ ID NO: 4:

(i) SEQUENZ CHARAKTERISTIKA:

(A) LÄNGE: 13 Aminosäuren

(B) ART: Aminosäure

(C) STRANGFORM: Einzel

(D) TOPOLOGIE: linear

(ii) ART DES MOLEKÜLS: Peptid

(v) ART DES FRAGMENTS: inneres
(vi) URSPRÜNLICHE HERKUNFT:

(A) ORGANISMUS: Bos taurus
(H) ZELLLINIE: Fötale Rinderherz Endothelzellinie

(xi) SEQUENZBESCHREIBUNG: SEQ ID NO: 4:

```
Xaa Pro Glu Phe Leu Leu Glu Gly Leu Ile Thr Asp Pro
1               5                   10
```

(2) INFORMATION ZU SEQ ID NO: 5:

(i) SEQUENZ CHARAKTERISTIKA:

(A) LÄNGE: 11 Aminosäuren
(B) ART: Aminosäure
(C) STRANGFORM: Einzel
(D) TOPOLOGIE: linear

(ii) ART DES MOLEKÜLS: Peptid
(v) ART DES FRAGMENTS: inneres
(vi) URSPRÜNLICHE HERKUNFT:

(A) ORGANISMUS: Bos taurus
(H) ZELLLINIE: Fötale Rinderherz Endothelzellinie

(xi) SEQUENZBESCHREIBUNG: SEQ ID NO: 5:

```
Xaa Gln Ala Glu Asn Val Ile Gln Val Xaa Gln
1               5                   10
```

(2) INFORMATION ZU SEQ ID NO: 6:

(i) SEQUENZ CHARAKTERISTIKA:

(A) LÄNGE: 16 Aminosäuren
(B) ART: Aminosäure
(C) STRANGFORM: Einzel
(D) TOPOLOGIE: linear

(ii) ART DES MOLEKÜLS: Peptid
(v) ART DES FRAGMENTS: inneres
(vi) URSPRÜNLICHE HERKUNFT:

(A) ORGANISMUS: Bos taurus
(H) ZELLLINIE: Fötale Rinderherz Endothelzellinie

(ix) MERKMALE:

(A) NAME/SCHLÜSSEL: Modified-site
(B) LAGE: 7
(D) SONSTIGE ANGABEN: /note= "Xaa = Ala oder Thr"

(xi) SEQUENZBESCHREIBUNG: SEQ ID NO: 6:

```
Val Glu Ile Val Phe Pro Xaa Gly Ile Leu Gln Ala Pro Phe Tyr Thr
1               5                   10                  15
```

(2) INFORMATION ZU SEQ ID NO: 7:

(i) SEQUENZ CHARAKTERISTIKA:

(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)
(xi) SEQUENZBESCHREIBUNG: SEQ ID NO: 7:

```
GGSCAYGARY TNACNCAYGC                                              20
```

(2) INFORMATION ZU SEQ ID NO: 8:

(i) SEQUENZ CHARAKTERISTIKA:

(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)
(xi) SEQUENZBESCHREIBUNG: SEQ ID NO: 8:

```
GARATYGTST TYCCYGCYGG                                              20
```

(2) INFORMATION ZU SEQ ID NO: 9:

(i) SEQUENZ CHARAKTERISTIKA:

(A) LÄNGE: 23 Basenpaare
(B) ART: Nukleinsäure
(C) STRANGFORM: Einzel
(D) TOPOLOGIE: linear

(ii) ART DES MOLEKÜLS: DNS (genomisch)
(xi) SEQUENZBESCHREIBUNG: SEQ ID NO: 9:

```
ATYCTSCAGG CYCCYTTYTA YAC                                          23
```

(2) INFORMATION ZU SEQ ID NO: 10:

(i) SEQUENZ CHARAKTERISTIKA:

(A) LÄNGE: 45 Basenpaare
(B) ART: Nukleinsäure
(C) STRANGFORM: Einzel
(D) TOPOLOGIE: linear

(ii) ART DES MOLEKÜLS: DNS (genomisch)
(xi) SEQUENZBESCHREIBUNG: SEQ ID NO: 10:

CGAGGGGGAT GGTCGACGGA AGCGACCTTT TTTTTTTTTT TTTTT 45

(2) INFORMATION ZU SEQ ID NO: 11:

(i) SEQUENZ CHARAKTERISTIKA:

(A) LÄNGE: 19 Basenpaare
(B) ART: Nukleinsäure
(C) STRANGFORM: Einzel
(D) TOPOLOGIE: linear

(ii) ART DES MOLEKÜLS: DNS (genomisch)
(xi) SEQUENZBESCHREIBUNG: SEQ ID NO: 11:

CGAGGGGGAT GGTCGACGG 19

(2) INFORMATION ZU SEQ ID NO: 12:

(i) SEQUENZ CHARAKTERISTIKA:

(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)
(xi) SEQUENZBESCHREIBUNG: SEQ ID NO: 12:

GATGGTCGAC GGAAGCGACC 20

(2) INFORMATION ZU SEQ ID NO: 13:

(i) SEQUENZ CHARAKTERISTIKA:

(A) LÄNGE: 570 Basenpaare
(B) ART: Nukleinsäure
(C) STRANGFORM: Einzel
(D) TOPOLOGIE: linear

(ii) ART DES MOLEKÜLS: cDNS zu mRNS
(vi) URSPRÜNLICHE HERKUNFT:

(A) ORGANISMUS: Bos taurus
(H) ZELLLINIE: Fötale Rinderherz Endothelzellinie

(ix) MERKMALE:

(A) NAME/SCHLÜSSEL: CDS
(B) LAGE: 1..567

(xi) SEQUENZBESCHREIBUNG: SEQ ID NO: 13:

```
ATC CTG CAG GCG CCA TTC TAC ACC CGC TCT TCA CCC AAT GCC TTA AAC      48
Ile Leu Gln Ala Pro Phe Tyr Thr Arg Ser Ser Pro Asn Ala Leu Asn
  1               5                  10                  15

TTC GGC GGC ATC GGC GTC GTC GTG GGC CAC GAG CTG ACT CAT GCT TTT      96
Phe Gly Gly Ile Gly Val Val Val Gly His Glu Leu Thr His Ala Phe
             20                  25                  30

GAT GAT CAA GGC CGA GAG TAC GAC AAG GAT GGG AAC CTC CGG CCC TGG     144
Asp Asp Gln Gly Arg Glu Tyr Asp Lys Asp Gly Asn Leu Arg Pro Trp
         35                  40                  45

TGG AAG AAC TCG TCC GTG GAG GCG TTC AAG CAG CAG ACC GCG TGC ATG     192
Trp Lys Asn Ser Ser Val Glu Ala Phe Lys Gln Gln Thr Ala Cys Met
     50                  55                  60

GTG GAG CAG TAC GGC AAC TAT AGC GTG AAC GGG GAG CCG GTG AAC GGC     240
Val Glu Gln Tyr Gly Asn Tyr Ser Val Asn Gly Glu Pro Val Asn Gly
 65                  70                  75                  80

CGG CAC ACC CTC GGC GAA AAC ATC GCC GAC AAC GGG GGC CTC AAG GCG     288
Arg His Thr Leu Gly Glu Asn Ile Ala Asp Asn Gly Gly Leu Lys Ala
                 85                  90                  95

GCC TAT CGG GCC TAC CAG AAC TGG GTC AAG AAG AAT GGG GCT GAG CAG     336
Ala Tyr Arg Ala Tyr Gln Asn Trp Val Lys Lys Asn Gly Ala Glu Gln
            100                 105                 110

ACA CTG CCC ACC CTG GGT CTC ACC AAC AAC CAG CTC TTC TTC CTG AGT     384
Thr Leu Pro Thr Leu Gly Leu Thr Asn Asn Gln Leu Phe Phe Leu Ser
        115                 120                 125

TTT GCA CAG GTC TGG TGT TCC GTC CGC ACC CCC GAG AGT TCG CAC GAA     432
Phe Ala Gln Val Trp Cys Ser Val Arg Thr Pro Glu Ser Ser His Glu
    130                 135                 140

GGT CTC ATC ACC GAT CCC CAC AGC CCC TCC CGC TTC CGG GTC ATC GGC     480
Gly Leu Ile Thr Asp Pro His Ser Pro Ser Arg Phe Arg Val Ile Gly
145                 150                 155                 160

TCC ATC TCC AAC TCC AAG GAG TTC TCG GAA CAC TTC CAC TGC CCG CCC     528
Ser Ile Ser Asn Ser Lys Glu Phe Ser Glu His Phe His Cys Pro Pro
                165                 170                 175

GGC TCA CCC ATG AAC CCG CAT CAC AAG TGT GAA GTC TGG TGA             570
Gly Ser Pro Met Asn Pro His His Lys Cys Glu Val Trp
            180                 185
```

(2) INFORMATION ZU SEQ ID NO: 14:

(i) SEQUENZ CHARAKTERISTIKA:

(A) LÄNGE: 189 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 14:

```
Ile Leu Gln Ala Pro Phe Tyr Thr Arg Ser Ser Pro Asn Ala Leu Asn
 1               5                  10                  15

Phe Gly Gly Ile Gly Val Val Val Gly His Glu Leu Thr His Ala Phe
            20                  25                  30

Asp Asp Gln Gly Arg Glu Tyr Asp Lys Asp Gly Asn Leu Arg Pro Trp
        35                  40                  45

Trp Lys Asn Ser Ser Val Glu Ala Phe Lys Gln Gln Thr Ala Cys Met
    50                  55                  60

Val Glu Gln Tyr Gly Asn Tyr Ser Val Asn Gly Glu Pro Val Asn Gly
65                  70                  75                  80

Arg His Thr Leu Gly Glu Asn Ile Ala Asp Asn Gly Gly Leu Lys Ala
                85                  90                  95

Ala Tyr Arg Ala Tyr Gln Asn Trp Val Lys Lys Asn Gly Ala Glu Gln
            100                 105                 110

Thr Leu Pro Thr Leu Gly Leu Thr Asn Asn Gln Leu Phe Phe Leu Ser
        115                 120                 125

Phe Ala Gln Val Trp Cys Ser Val Arg Thr Pro Glu Ser Ser His Glu
    130                 135                 140

Gly Leu Ile Thr Asp Pro His Ser Pro Ser Arg Phe Arg Val Ile Gly
145                 150                 155                 160

Ser Ile Ser Asn Ser Lys Glu Phe Ser Glu His Phe His Cys Pro Pro
                165                 170                 175

Gly Ser Pro Met Asn Pro His His Lys Cys Glu Val Trp
            180                 185
```

(2) INFORMATION ZU SEQ ID NO: 15:

(i) SEQUENZ CHARAKTERISTIKA:

(A) LÄNGE: 27 Basenpaare
(B) ART: Nukleinsäure
(C) STRANGFORM: Einzel

(D) TOPOLOGIE: linear

(ii) ART DES MOLEKÜLS: DNS (genomisch)
(iii) HYPOTHETISCH: JA
(xi) SEQUENZBESCHREIBUNG: SEQ ID NO: 15:

GAGAGAGAGT CGACGGTACC NNNNNNN 27

(2) INFORMATION ZU SEQ ID NO: 16:

(i) SEQUENZ CHARAKTERISTIKA:

(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)
(xi) SEQUENZBESCHREIBUNG: SEQ IP NO: 16:

CGGCCCTGGT GGAAGAACTC G 21

(2) INFORMATION ZU SEQ ID NO: 17:

(i) SEQUENZ CHARAKTERISTIKA:

(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)
(iii) ANTISENSE: JA
(xi) SEQUENZBESCHREIBUNG: SEQ ID NO: 17:

TGCGGACGGA ACACCAGACC T 21

(2) INFORMATION ZU SEQ ID NO: 18:

(i) SEQUENZ CHARAKTERISTIKA:

(A) LÄNGE: 1703 Basenpaare
(B) ART: Nukleinsäure
(C) STRANGFORM: Einzel
(D) TOPOLOGIE: linear

(ii) ART DES MOLEKÜLS: cDNS zu mRNS
(vi) URSPRÜNLICHE HERKUNFT:

(A) ORGANISMUS: Bos taurus
(H) ZELLLINIE: Fötale Rinderherz Endothelzellinie

(ix) MERKMALE:

(A) NAME/SCHLÜSSEL: CDS
(B) LAGE: 2..1703

(xi) SEQUENZBESCHREIBUNG: SEQ ID NO: 18:

```
T GGC CAC TCG CGC TGG GGG ACC TTC AGC AAC CTC TGG GAA CAC AAC      46
  Gly His Ser Arg Trp Gly Thr Phe Ser Asn Leu Trp Glu His Asn
    1               5                  10                  15

CAA GCC ATC ATC AAG CAC CTC CTT GAA AAC TCC ACG GCC AGC GTG AGC    94
Gln Ala Ile Ile Lys His Leu Leu Glu Asn Ser Thr Ala Ser Val Ser
                 20                  25                  30

GAG GCA GAG AGG AAG GAC CAG GAG TAC TAC CGA GCC TGC ATG AAC GAA   142
Glu Ala Glu Arg Lys Asp Gln Glu Tyr Tyr Arg Ala Cys Met Asn Glu
             35                  40                  45

ACC AGG ATT GAG GAG CTC AAG GCC AAA CCC CTG ATG GAG CTC ATT GAG   190
Thr Arg Ile Glu Glu Leu Lys Ala Lys Pro Leu Met Glu Leu Ile Glu
         50                  55                  60

AAG CTC GGC GGC TGG AAC ATC ACG GGG CCC TGG GAC AAG GAC AAC TTC   238
Lys Leu Gly Gly Trp Asn Ile Thr Gly Pro Trp Asp Lys Asp Asn Phe
     65                  70                  75

CAG GAC ACC CTG CAG GTG GTC ACA TCC CAC TAC CAC ACC TCC CCC TTC   286
Gln Asp Thr Leu Gln Val Val Thr Ser His Tyr His Thr Ser Pro Phe
 80                  85                  90                  95

TTC TCC GTC TAC GTC AGT GCC GAC TCC AAG AAT TCC AAC AGC AAC GTG   334
Phe Ser Val Tyr Val Ser Ala Asp Ser Lys Asn Ser Asn Ser Asn Val
                100                 105                 110

ATC CAA GTG GAC CAG TCT GGC CTG GGC TTA CCC TCA AGA GAT TAT TAC   382
Ile Gln Val Asp Gln Ser Gly Leu Gly Leu Pro Ser Arg Asp Tyr Tyr
            115                 120                 125

CTG AAC AAA ACC GAG AAT GAG AAG GTG CTG ACG GGA TAC CTG AAC TAC   430
Leu Asn Lys Thr Glu Asn Glu Lys Val Leu Thr Gly Tyr Leu Asn Tyr
        130                 135                 140

ATG GTC CAG CTG GGG AAG CTG CTG GGA GGA GGG GCC GAG GAC ACC ATC   478
Met Val Gln Leu Gly Lys Leu Leu Gly Gly Gly Ala Glu Asp Thr Ile
    145                 150                 155
```

```
CGG CCC CAG ATG CAG CAG ATC CTG GAC TTT GAG ACG GCG CTG GCC AAC      526
Arg Pro Gln Met Gln Gln Ile Leu Asp Phe Glu Thr Ala Leu Ala Asn
160                 165                 170                 175

ATC ACC ATC CCC CAG GAG AAG CGC CGG GAC GAG GAA CTC ATC TAC CAC      574
Ile Thr Ile Pro Gln Glu Lys Arg Arg Asp Glu Glu Leu Ile Tyr His
                180                 185                 190

AAA GTG ACG GCG GCT GAG TTG CAG ACC TTG GCG CCC GCC ATC AAC TGG      622
Lys Val Thr Ala Ala Glu Leu Gln Thr Leu Ala Pro Ala Ile Asn Trp
            195                 200                 205

CTG CCC TTC CTC AAC ACC ATC TTC TAC CCC GTG GAG ATC AAT GAA TCA      670
Leu Pro Phe Leu Asn Thr Ile Phe Tyr Pro Val Glu Ile Asn Glu Ser
        210                 215                 220

GAG CCT ATT GTC ATC TAC GAC AAA GAA TAC CTG AGC AAG GTC TCC ACC      718
Glu Pro Ile Val Ile Tyr Asp Lys Glu Tyr Leu Ser Lys Val Ser Thr
    225                 230                 235

CTC ATC AAC AGC ACA GAC AAA TGC CTG CTG AAC AAC TAC ATG ATC TGG      766
Leu Ile Asn Ser Thr Asp Lys Cys Leu Leu Asn Asn Tyr Met Ile Trp
240                 245                 250                 255

AAC CTG GTA CGG AAG ACG AGC TCC TTC CTC GAT CAG CGC TTC CAG GAC      814
Asn Leu Val Arg Lys Thr Ser Ser Phe Leu Asp Gln Arg Phe Gln Asp
                260                 265                 270

GCC GAC GAG AAG TTC ATG GAA GTC ATG TAT GGG ACC AAG AAG ACG TGT      862
Ala Asp Glu Lys Phe Met Glu Val Met Tyr Gly Thr Lys Lys Thr Cys
            275                 280                 285

CTT CCC CGC TGG AAG TTT TGT GTG AGT GAT ACA GAG AAC ACC TTG GGC      910
Leu Pro Arg Trp Lys Phe Cys Val Ser Asp Thr Glu Asn Thr Leu Gly
        290                 295                 300

TTC GCC CTG GGC CCC ATG TTC GTC AAA GCG ACC TTC GCT GAG GAC AGC      958
Phe Ala Leu Gly Pro Met Phe Val Lys Ala Thr Phe Ala Glu Asp Ser
    305                 310                 315

AAG AAC ATA GCC AGC GAG ATC ATC CTG GAG ATC AAG AAG GCG TTT GAA     1006
Lys Asn Ile Ala Ser Glu Ile Ile Leu Glu Ile Lys Lys Ala Phe Glu
320                 325                 330                 335

GAG AGC CTG AGC ACC CTG AAG TGG ATG GAT GAA GAT ACT CGG AAA TCG     1054
Glu Ser Leu Ser Thr Leu Lys Trp Met Asp Glu Asp Thr Arg Lys Ser
                340                 345                 350

GCC AAG GAA AAG GCG GAC GCG ATC TAC AAC ATG ATA GGC TAC CCC AAC     1102
Ala Lys Glu Lys Ala Asp Ala Ile Tyr Asn Met Ile Gly Tyr Pro Asn
            355                 360                 365
```

```
TTT ATC ATG GAC CCC AAG GAG CTG GAC AAA GTG TTC AAT GAC TAC ACC      1150
Phe Ile Met Asp Pro Lys Glu Leu Asp Lys Val Phe Asn Asp Tyr Thr
        370                 375                     380

GCT GTG CCA GAC CTC TAC TTC GAG AAC GCC ATG CGG TTT TTC AAC TTC      1198
Ala Val Pro Asp Leu Tyr Phe Glu Asn Ala Met Arg Phe Phe Asn Phe
    385                 390                     395

TCC TGG AGG GTC ACT GCC GAC CAG CTC CGG AAA GCG CCC AAC AGA GAT      1246
Ser Trp Arg Val Thr Ala Asp Gln Leu Arg Lys Ala Pro Asn Arg Asp
400                 405                     410                 415

CAG TGG AGC ATG ACC CCG CCC ATG GTG AAC GCC TAC TAC TCG CCC ACC      1294
Gln Trp Ser Met Thr Pro Pro Met Val Asn Ala Tyr Tyr Ser Pro Thr
                420                     425                 430

AAG AAC GAG ATC GTG TTT CCG GCC GGA ATC CTG CAG GCG CCA TTC TAC      1342
Lys Asn Glu Ile Val Phe Pro Ala Gly Ile Leu Gln Ala Pro Phe Tyr
            435                 440                     445

ACC CGC TCT TCA CCC AAT GCC TTA AAC TTC GGC GGC ATC GGC GTC GTC      1390
Thr Arg Ser Ser Pro Asn Ala Leu Asn Phe Gly Gly Ile Gly Val Val
        450                 455                     460

GTG GGC CAC GAG CTG ACT CAT GCT TTT GAT GAT CAA GGC CGA GAG TAC      1438
Val Gly His Glu Leu Thr His Ala Phe Asp Asp Gln Gly Arg Glu Tyr
    465                 470                     475

GAC AAG GAT GGG AAC CTC CGG CCC TGG TGG AAG AAC TCG TCC GTG GAG      1486
Asp Lys Asp Gly Asn Leu Arg Pro Trp Trp Lys Asn Ser Ser Val Glu
480                 485                     490                 495

GCG TTC AAG CAG CAG ACC GCG TGC ATG GTG GAG CAG TAC GGC AAC TAT      1534
Ala Phe Lys Gln Gln Thr Ala Cys Met Val Glu Gln Tyr Gly Asn Tyr
                500                     505                 510

AGC GTG AAC GGG GAG CCG GTG AAC GGC CGG CAC ACC CTC GGC GAA AAC      1582
Ser Val Asn Gly Glu Pro Val Asn Gly Arg His Thr Leu Gly Glu Asn
            515                 520                     525

ATC GCC GAC AAC GGG GGC CTC AAG GCG GCC TAT CGG GCC TAC CAG AAC      1630
Ile Ala Asp Asn Gly Gly Leu Lys Ala Ala Tyr Arg Ala Tyr Gln Asn
        530                 535                     540

TGG GTC AAG AAG AAT GGG GCT GAG CAG ACA CTG CCC ACC CTG GGT CTC      1678
Trp Val Lys Lys Asn Gly Ala Glu Gln Thr Leu Pro Thr Leu Gly Leu
    545                 550                     555

ACC AAC AAC CAG CTC TTC TTC CTG A                                    1703
Thr Asn Asn Gln Leu Phe Phe Leu
560                 565
```

(2) INFORMATION ZU SEQ ID NO: 19:

(i) SEQUENZ CHARAKTERISTIKA:

(A) LÄNGE: 567 Aminosäuren

(B) ART: Aminosäure
(D) TOPOLOGIE: linear

(ii) ART DES MOLEKÜLS: Protein
(xi) SEQUENZBESCHREIBUNG: SEQ ID NO: 19:

```
Gly His Ser Arg Trp Gly Thr Phe Ser Asn Leu Trp Glu His Asn Gln
 1               5                  10                  15

Ala Ile Ile Lys His Leu Leu Glu Asn Ser Thr Ala Ser Val Ser Glu
            20                  25                  30

Ala Glu Arg Lys Asp Gln Glu Tyr Tyr Arg Ala Cys Met Asn Glu Thr
        35                  40                  45

Arg Ile Glu Glu Leu Lys Ala Lys Pro Leu Met Glu Leu Ile Glu Lys
    50                  55                  60

Leu Gly Gly Trp Asn Ile Thr Gly Pro Trp Asp Lys Asp Asn Phe Gln
65                  70                  75                  80

Asp Thr Leu Gln Val Val Thr Ser His Tyr His Thr Ser Pro Phe Phe
                85                  90                  95

Ser Val Tyr Val Ser Ala Asp Ser Lys Asn Ser Asn Ser Asn Val Ile
            100                 105                 110

Gln Val Asp Gln Ser Gly Leu Gly Leu Pro Ser Arg Asp Tyr Tyr Leu
        115                 120                 125

Asn Lys Thr Glu Asn Glu Lys Val Leu Thr Gly Tyr Leu Asn Tyr Met
    130                 135                 140

Val Gln Leu Gly Lys Leu Leu Gly Gly Gly Ala Glu Asp Thr Ile Arg
145                 150                 155                 160

Pro Gln Met Gln Gln Ile Leu Asp Phe Glu Thr Ala Leu Ala Asn Ile
                165                 170                 175

Thr Ile Pro Gln Glu Lys Arg Arg Asp Glu Glu Leu Ile Tyr His Lys
            180                 185                 190

Val Thr Ala Ala Glu Leu Gln Thr Leu Ala Pro Ala Ile Asn Trp Leu
        195                 200                 205

Pro Phe Leu Asn Thr Ile Phe Tyr Pro Val Glu Ile Asn Glu Ser Glu
    210                 215                 220
```

Pro Ile Val Ile Tyr Asp Lys Glu Tyr Leu Ser Lys Val Ser Thr Leu
225 230 235 240

Ile Asn Ser Thr Asp Lys Cys Leu Leu Asn Asn Tyr Met Ile Trp Asn
245 250 255

Leu Val Arg Lys Thr Ser Ser Phe Leu Asp Gln Arg Phe Gln Asp Ala
260 265 270

Asp Glu Lys Phe Met Glu Val Met Tyr Gly Thr Lys Lys Thr Cys Leu
275 280 285

Pro Arg Trp Lys Phe Cys Val Ser Asp Thr Glu Asn Thr Leu Gly Phe
290 295 300

Ala Leu Gly Pro Met Phe Val Lys Ala Thr Phe Ala Glu Asp Ser Lys
305 310 315 320

Asn Ile Ala Ser Glu Ile Ile Leu Glu Ile Lys Lys Ala Phe Glu Glu
325 330 335

Ser Leu Ser Thr Leu Lys Trp Met Asp Glu Asp Thr Arg Lys Ser Ala
340 345 350

Lys Glu Lys Ala Asp Ala Ile Tyr Asn Met Ile Gly Tyr Pro Asn Phe
355 360 365

Ile Met Asp Pro Lys Glu Leu Asp Lys Val Phe Asn Asp Tyr Thr Ala
370 375 380

Val Pro Asp Leu Tyr Phe Glu Asn Ala Met Arg Phe Phe Asn Phe Ser
385 390 395 400

Trp Arg Val Thr Ala Asp Gln Leu Arg Lys Ala Pro Asn Arg Asp Gln
405 410 415

Trp Ser Met Thr Pro Pro Met Val Asn Ala Tyr Tyr Ser Pro Thr Lys
420 425 430

Asn Glu Ile Val Phe Pro Ala Gly Ile Leu Gln Ala Pro Phe Tyr Thr
435 440 445

Arg Ser Ser Pro Asn Ala Leu Asn Phe Gly Gly Ile Gly Val Val Val
450 455 460

Gly His Glu Leu Thr His Ala Phe Asp Asp Gln Gly Arg Glu Tyr Asp
465 470 475 480

Lys Asp Gly Asn Leu Arg Pro Trp Trp Lys Asn Ser Ser Val Glu Ala
485 490 495

Phe Lys Gln Gln Thr Ala Cys Met Val Glu Gln Tyr Gly Asn Tyr Ser
500 505 510

Val Asn Gly Glu Pro Val Asn Gly Arg His Thr Leu Gly Glu Asn Ile
515 520 525

Ala Asp Asn Gly Gly Leu Lys Ala Ala Tyr Arg Ala Tyr Gln Asn Trp
530 535 540

Val Lys Lys Asn Gly Ala Glu Gln Thr Leu Pro Thr Leu Gly Leu Thr
545 550 555 560

Asn Asn Gln Leu Phe Phe Leu
565

(2) INFORMATION ZU SEQ ID NO: 20:

(i) SEQUENZ CHARAKTERISTIKA:

(A) LÄNGE: 23 Basenpaare
(B) ART: Nukleinsäure
(C) STRANGFORM: Einzel
(D) TOPOLOGIE: linear

(ii) ART DES MOLEKÜLS: DNS (genomisch)
(iii) ANTISENSE: JA
(xi) SEQUENZBESCHREIBUNG: SEQ ID NO: 20:

GCCAGCGCCG TCTCAAAGTC CAG 23

(2) INFORMATION ZU SEQ ID NO: 21:

(i) SEQUENZ CHARAKTERISTIKA:

(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)
(iii) ANTISENSE: NEIN
(xi) SEQUENZBESCHREIBUNG: SEQ ID NO: 21:

TGGGGGACCT TCAGCAACCT CT 22

(2) INFORMATION ZU SEQ ID NO: 22:

(i) SEQUENZ CHARAKTERISTIKA:

(A) LÄNGE: 2129 Basenpaare
(B) ART: Nukleinsäure
(C) STRANGFORM: Einzel
(D) TOPOLOGIE: linear

(ii) ART DES MOLEKÜLS: cDNS zu mRNS
(vi) URSPRÜNLICHE HERKUNFT:

(A) ORGANISMUS: Bos taurus
(H) ZELLLINIE: Fötale Rinderherz Endothelzellinie

(ix) MERKMALE:

(A) NAME/SCHLÜSSEL: CDS
(B) LAGE: 3..2126

(xi) SEQUENZBESCHREIBUNG: SEQ ID NO: 22:

```
GG ACC CCG GTG GAG AAG CGG CTG GTG GTG CTG GTG GCG CTC CTG GCG          47
   Thr Pro Val Glu Lys Arg Leu Val Val Leu Val Ala Leu Leu Ala
    1               5                  10                  15

GCG GCA TTG GTG GCC TGT TTG GCA GTA CTG GGC ATC CAA TAC CAG ACA         95
Ala Ala Leu Val Ala Cys Leu Ala Val Leu Gly Ile Gln Tyr Gln Thr
                20                  25                  30

AGA ACG CCC TCG GTG TGC CTA AGT GAG GCC TGC ATC TCG GTG ACC AGC        143
Arg Thr Pro Ser Val Cys Leu Ser Glu Ala Cys Ile Ser Val Thr Ser
            35                  40                  45

TCC ATC TTG AGT TCC ATG GAC CCC ACG GTG GAC CCC TGC CAG GAC TTC        191
Ser Ile Leu Ser Ser Met Asp Pro Thr Val Asp Pro Cys Gln Asp Phe
        50                  55                  60

TTC ACC TAT GCC TGT GGC GGC TGG ATC AAA GCC AAC CCC GTG CCG GAT        239
Phe Thr Tyr Ala Cys Gly Gly Trp Ile Lys Ala Asn Pro Val Pro Asp
    65                  70                  75

GGC CAC TCG CGC TGG GGG ACC TTC AGC AAC CTC TGG GAA CAC AAC CAA        287
Gly His Ser Arg Trp Gly Thr Phe Ser Asn Leu Trp Glu His Asn Gln
80                  85                  90                  95

GCC ATC ATC AAG CAC CTC CTT GAA AAC TCC ACG GCC AGC GTG AGC GAG        335
Ala Ile Ile Lys His Leu Leu Glu Asn Ser Thr Ala Ser Val Ser Glu
                100                 105                 110

GCA GAG AGG AAG GAC CAG GAG TAC TAC CGA GCC TGC ATG AAC GAA ACC        383
Ala Glu Arg Lys Asp Gln Glu Tyr Tyr Arg Ala Cys Met Asn Glu Thr
            115                 120                 125

AGG ATT GAG GAG CTC AAG GCC AAA CCC CTG ATG GAG CTC ATT GAG AAG        431
Arg Ile Glu Glu Leu Lys Ala Lys Pro Leu Met Glu Leu Ile Glu Lys
        130                 135                 140

CTC GGC GGC TGG AAC ATC ACG GGG CCC TGG GAC AAG GAC AAC TTC CAG        479
Leu Gly Gly Trp Asn Ile Thr Gly Pro Trp Asp Lys Asp Asn Phe Gln
    145                 150                 155

GAC ACC CTG CAG GTG GTC ACA TCC CAC TAC CAC ACC TCC CCC TTC TTC        527
Asp Thr Leu Gln Val Val Thr Ser His Tyr His Thr Ser Pro Phe Phe
160                 165                 170                 175
```

TCC GTC TAC GTC AGT GCC GAC TCC AAG AAT TCC AAC AGC AAC GTG ATC 575
Ser Val Tyr Val Ser Ala Asp Ser Lys Asn Ser Asn Ser Asn Val Ile
180 185 190

CAA GTG GAC CAG TCT GGC CTG GGC TTA CCC TCA AGA GAT TAT TAC CTG 623
Gln Val Asp Gln Ser Gly Leu Gly Leu Pro Ser Arg Asp Tyr Tyr Leu
195 200 205

AAC AAA ACC GAG AAT GAG AAG GTG CTG ACG GGA TAC CTG AAC TAC ATG 671
Asn Lys Thr Glu Asn Glu Lys Val Leu Thr Gly Tyr Leu Asn Tyr Met
210 215 220

GTC CAG CTG GGG AAG CTG CTG GGA GGA GGG GCC GAG GAC ACC ATC CGG 719
Val Gln Leu Gly Lys Leu Leu Gly Gly Gly Ala Glu Asp Thr Ile Arg
225 230 235

CCC CAG ATG CAG CAG ATC CTG GAC TTT GAG ACG GCG CTG GCC AAC ATC 767
Pro Gln Met Gln Gln Ile Leu Asp Phe Glu Thr Ala Leu Ala Asn Ile
240 245 250 255

ACC ATC CCC CAG GAG AAG CGC CGG GAC GAG GAA CTC ATC TAC CAC AAA 815
Thr Ile Pro Gln Glu Lys Arg Arg Asp Glu Glu Leu Ile Tyr His Lys
260 265 270

GTG ACG GCG GCT GAG TTG CAG ACC TTG GCG CCC GCC ATC AAC TGG CTG 863
Val Thr Ala Ala Glu Leu Gln Thr Leu Ala Pro Ala Ile Asn Trp Leu
275 280 285

CCC TTC CTC AAC ACC ATC TTC TAC CCC GTG GAG ATC AAT GAA TCA GAG 911
Pro Phe Leu Asn Thr Ile Phe Tyr Pro Val Glu Ile Asn Glu Ser Glu
290 295 300

CCT ATT GTC ATC TAC GAC AAA GAA TAC CTG AGC AAG GTC TCC ACC CTC 959
Pro Ile Val Ile Tyr Asp Lys Glu Tyr Leu Ser Lys Val Ser Thr Leu
305 310 315

ATC AAC AGC ACA GAC AAA TGC CTG CTG AAC AAC TAC ATG ATC TGG AAC 1007
Ile Asn Ser Thr Asp Lys Cys Leu Leu Asn Asn Tyr Met Ile Trp Asn
320 325 330 335

CTG GTA CGG AAG ACG AGC TCC TTC CTC GAT CAG CGC TTC CAG GAC GCC 1055
Leu Val Arg Lys Thr Ser Ser Phe Leu Asp Gln Arg Phe Gln Asp Ala
340 345 350

GAC GAG AAG TTC ATG GAA GTC ATG TAT GGG ACC AAG AAG ACG TGT CTT 1103
Asp Glu Lys Phe Met Glu Val Met Tyr Gly Thr Lys Lys Thr Cys Leu
355 360 365

CCC CGC TGG AAG TTT TGT GTG AGT GAT ACA GAG AAC ACC TTG GGC TTC 1151
Pro Arg Trp Lys Phe Cys Val Ser Asp Thr Glu Asn Thr Leu Gly Phe
370 375 380

```
GCC CTG GGC CCC ATG TTC GTC AAA GCG ACC TTC GCT GAG GAC AGC AAG      1199
Ala Leu Gly Pro Met Phe Val Lys Ala Thr Phe Ala Glu Asp Ser Lys
    385                 390                 395

AAC ATA GCC AGC GAG ATC ATC CTG GAG ATC AAG AAG GCG TTT GAA GAG      1247
Asn Ile Ala Ser Glu Ile Ile Leu Glu Ile Lys Lys Ala Phe Glu Glu
400                 405                 410                 415

AGC CTG AGC ACC CTG AAG TGG ATG GAT GAA GAT ACT CGG AAA TCG GCC      1295
Ser Leu Ser Thr Leu Lys Trp Met Asp Glu Asp Thr Arg Lys Ser Ala
                420                 425                 430

AAG GAA AAG GCG GAC GCG ATC TAC AAC ATG ATA GGC TAC CCC AAC TTT      1343
Lys Glu Lys Ala Asp Ala Ile Tyr Asn Met Ile Gly Tyr Pro Asn Phe
            435                 440                 445

ATC ATG GAC CCC AAG GAG CTG GAC AAA GTG TTC AAT GAC TAC ACC GCT      1391
Ile Met Asp Pro Lys Glu Leu Asp Lys Val Phe Asn Asp Tyr Thr Ala
        450                 455                 460

GTG CCA GAC CTC TAC TTC GAG AAC GCC ATG CGG TTT TTC AAC TTC TCC      1439
Val Pro Asp Leu Tyr Phe Glu Asn Ala Met Arg Phe Phe Asn Phe Ser
    465                 470                 475

TGG AGG GTC ACT GCC GAC CAG CTC CGG AAA GCG CCC AAC AGA GAT CAG      1487
Trp Arg Val Thr Ala Asp Gln Leu Arg Lys Ala Pro Asn Arg Asp Gln
480                 485                 490                 495

TGG AGC ATG ACC CCG CCC ATG GTG AAC GCC TAC TAC TCG CCC ACC AAG      1535
Trp Ser Met Thr Pro Pro Met Val Asn Ala Tyr Tyr Ser Pro Thr Lys
                500                 505                 510

AAC GAG ATC GTG TTT CCG GCC GGA ATC CTG CAG GCG CCA TTC TAC ACC      1583
Asn Glu Ile Val Phe Pro Ala Gly Ile Leu Gln Ala Pro Phe Tyr Thr
            515                 520                 525

CGC TCT TCA CCC AAT GCC TTA AAC TTC GGC GGC ATC GGC GTC GTC GTG      1631
Arg Ser Ser Pro Asn Ala Leu Asn Phe Gly Gly Ile Gly Val Val Val
        530                 535                 540

GGC CAC GAG CTG ACT CAT GCT TTT GAT GAT CAA GGC CGA GAG TAC GAC      1679
Gly His Glu Leu Thr His Ala Phe Asp Asp Gln Gly Arg Glu Tyr Asp
    545                 550                 555

AAG GAT GGG AAC CTC CGG CCC TGG TGG AAG AAC TCG TCC GTG GAG GCG      1727
Lys Asp Gly Asn Leu Arg Pro Trp Trp Lys Asn Ser Ser Val Glu Ala
560                 565                 570                 575

TTC AAG CAG CAG ACC GCG TGC ATG GTG GAG CAG TAC GGC AAC TAT AGC      1775
Phe Lys Gln Gln Thr Ala Cys Met Val Glu Gln Tyr Gly Asn Tyr Ser
                580                 585                 590
```

```
GTG AAC GGG GAG CCG GTG AAC GGC CGG CAC ACC CTC GGC GAA AAC ATC        1823
Val Asn Gly Glu Pro Val Asn Gly Arg His Thr Leu Gly Glu Asn Ile
            595                 600                 605

GCC GAC AAC GGG GGC CTC AAG GCG GCC TAT CGG GCC TAC CAG AAC TGG        1871
Ala Asp Asn Gly Gly Leu Lys Ala Ala Tyr Arg Ala Tyr Gln Asn Trp
        610                 615                 620

GTC AAG AAG AAT GGG GCT GAG CAG ACA CTG CCC ACC CTG GGT CTC ACC        1919
Val Lys Lys Asn Gly Ala Glu Gln Thr Leu Pro Thr Leu Gly Leu Thr
    625                 630                 635

AAC AAC CAG CTC TTC TTC CTG AGT TTT GCA CAG GTC TGG TGT TCC GTC        1967
Asn Asn Gln Leu Phe Phe Leu Ser Phe Ala Gln Val Trp Cys Ser Val
640                 645                 650                 655

CGC ACC CCC GAG AGT TCG CAC GAA GGT CTC ATC ACC GAT CCC CAC AGC        2015
Arg Thr Pro Glu Ser Ser His Glu Gly Leu Ile Thr Asp Pro His Ser
                660                 665                 670

CCC TCC CGC TTC CGG GTC ATC GGC TCC ATC TCC AAC TCC AAG GAG TTC        2063
Pro Ser Arg Phe Arg Val Ile Gly Ser Ile Ser Asn Ser Lys Glu Phe
            675                 680                 685

TCG GAA CAC TTC CAC TGC CCG CCC GGC TCA CCC ATG AAC CCG CAT CAC        2111
Ser Glu His Phe His Cys Pro Pro Gly Ser Pro Met Asn Pro His His
        690                 695                 700

AAG TGT GAA GTC TGG TGA                                                2129
Lys Cys Glu Val Trp
    705
```

(2) INFORMATION ZU SEQ ID NO: 23:

(i) SEQUENZ CHARAKTERISTIKA:

(A) LÄNGE: 708 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 23:

```
Thr Pro Val Glu Lys Arg Leu Val Val Leu Val Ala Leu Leu Ala Ala
 1               5                  10                  15

Ala Leu Val Ala Cys Leu Ala Val Leu Gly Ile Gln Tyr Gln Thr Arg
            20                  25                  30

Thr Pro Ser Val Cys Leu Ser Glu Ala Cys Ile Ser Val Thr Ser Ser
        35                  40                  45
```

Ile Leu Ser Ser Met Asp Pro Thr Val Asp Pro Cys Gln Asp Phe Phe
50 55 60

Thr Tyr Ala Cys Gly Gly Trp Ile Lys Ala Asn Pro Val Pro Asp Gly
65 70 75 80

His Ser Arg Trp Gly Thr Phe Ser Asn Leu Trp Glu His Asn Gln Ala
85 90 95

Ile Ile Lys His Leu Leu Glu Asn Ser Thr Ala Ser Val Ser Glu Ala
100 105 110

Glu Arg Lys Asp Gln Glu Tyr Tyr Arg Ala Cys Met Asn Glu Thr Arg
115 120 125

Ile Glu Glu Leu Lys Ala Lys Pro Leu Met Glu Leu Ile Glu Lys Leu
130 135 140

Gly Gly Trp Asn Ile Thr Gly Pro Trp Asp Lys Asp Asn Phe Gln Asp
145 150 155 160

Thr Leu Gln Val Val Thr Ser His Tyr His Thr Ser Pro Phe Phe Ser
165 170 175

Val Tyr Val Ser Ala Asp Ser Lys Asn Ser Asn Ser Asn Val Ile Gln
180 185 190

Val Asp Gln Ser Gly Leu Gly Leu Pro Ser Arg Asp Tyr Tyr Leu Asn
195 200 205

Lys Thr Glu Asn Glu Lys Val Leu Thr Gly Tyr Leu Asn Tyr Met Val
210 215 220

Gln Leu Gly Lys Leu Leu Gly Gly Gly Ala Glu Asp Thr Ile Arg Pro
225 230 235 240

Gln Met Gln Gln Ile Leu Asp Phe Glu Thr Ala Leu Ala Asn Ile Thr
245 250 255

Ile Pro Gln Glu Lys Arg Arg Asp Glu Glu Leu Ile Tyr His Lys Val
260 265 270

Thr Ala Ala Glu Leu Gln Thr Leu Ala Pro Ala Ile Asn Trp Leu Pro
275 280 285

Phe Leu Asn Thr Ile Phe Tyr Pro Val Glu Ile Asn Glu Ser Glu Pro
290 295 300

Ile Val Ile Tyr Asp Lys Glu Tyr Leu Ser Lys Val Ser Thr Leu Ile
305 310 315 320

Asn Ser Thr Asp Lys Cys Leu Leu Asn Asn Tyr Met Ile Trp Asn Leu
325 330 335

Val Arg Lys Thr Ser Ser Phe Leu Asp Gln Arg Phe Gln Asp Ala Asp
340 345 350

Glu Lys Phe Met Glu Val Met Tyr Gly Thr Lys Lys Thr Cys Leu Pro
355 360 365

Arg Trp Lys Phe Cys Val Ser Asp Thr Glu Asn Thr Leu Gly Phe Ala
370 375 380

Leu Gly Pro Met Phe Val Lys Ala Thr Phe Ala Glu Asp Ser Lys Asn
385 390 395 400

Ile Ala Ser Glu Ile Ile Leu Glu Ile Lys Lys Ala Phe Glu Glu Ser
405 410 415

Leu Ser Thr Leu Lys Trp Met Asp Glu Asp Thr Arg Lys Ser Ala Lys
420 425 430

Glu Lys Ala Asp Ala Ile Tyr Asn Met Ile Gly Tyr Pro Asn Phe Ile
435 440 445

Met Asp Pro Lys Glu Leu Asp Lys Val Phe Asn Asp Tyr Thr Ala Val
450 455 460

Pro Asp Leu Tyr Phe Glu Asn Ala Met Arg Phe Phe Asn Phe Ser Trp
465 470 475 480

Arg Val Thr Ala Asp Gln Leu Arg Lys Ala Pro Asn Arg Asp Gln Trp
485 490 495

Ser Met Thr Pro Pro Met Val Asn Ala Tyr Tyr Ser Pro Thr Lys Asn
500 505 510

Glu Ile Val Phe Pro Ala Gly Ile Leu Gln Ala Pro Phe Tyr Thr Arg
515 520 525

Ser Ser Pro Asn Ala Leu Asn Phe Gly Gly Ile Gly Val Val Val Gly
530 535 540

His Glu Leu Thr His Ala Phe Asp Asp Gln Gly Arg Glu Tyr Asp Lys
545 550 555 560

Asp Gly Asn Leu Arg Pro Trp Trp Lys Asn Ser Ser Val Glu Ala Phe
565 570 575

Lys Gln Gln Thr Ala Cys Met Val Glu Gln Tyr Gly Asn Tyr Ser Val
580 585 590

Asn Gly Glu Pro Val Asn Gly Arg His Thr Leu Gly Glu Asn Ile Ala
595 600 605

Asp Asn Gly Gly Leu Lys Ala Ala Tyr Arg Ala Tyr Gln Asn Trp Val
610 615 620

Lys Lys Asn Gly Ala Glu Gln Thr Leu Pro Thr Leu Gly Leu Thr Asn
625 630 635 640

Asn Gln Leu Phe Phe Leu Ser Phe Ala Gln Val Trp Cys Ser Val Arg
645 650 655

Thr Pro Glu Ser Ser His Glu Gly Leu Ile Thr Asp Pro His Ser Pro
660 665 670

Ser Arg Phe Arg Val Ile Gly Ser Ile Ser Asn Ser Lys Glu Phe Ser
675 680 685

Glu His Phe His Cys Pro Pro Gly Ser Pro Met Asn Pro His His Lys
690 695 700

Cys Glu Val Trp
705

(2) INFORMATION ZU SEQ ID NO: 24:

(i) SEQUENZ CHARAKTERISTIKA:

(A) LÄNGE: 2533 Basenpaare
(B) ART: Nukleinsäure
(C) STRANGFORM: Einzel
(D) TOPOLOGIE: linear

(ii) ART DES MOLEKÜLS: cDNS zu mRNS
(vi) URSPRÜNLICHE HERKUNFT:

(A) ORGANISMUS: Homo sapiens
(F) GEWEBETYP: Placenta

(ix) MERKMALE:

(A) NAME/SCHLÜSSEL: CDS
(B) LAGE: 1..2109

(xi) SEQUENZBESCHREIBUNG: SEQ ID NO: 24:

```
CGG CTG GTG GTG TTG GTG GTA CTT CTG GCG GCA GGA CTG GTG GCC TGC         48
Arg Leu Val Val Leu Val Val Leu Leu Ala Ala Gly Leu Val Ala Cys
  1               5                  10                  15

TTG GCA GCA CTG GGC ATC CAG TAC CAG ACA AGA TCC CCC TCT GTG TGC         96
Leu Ala Ala Leu Gly Ile Gln Tyr Gln Thr Arg Ser Pro Ser Val Cys
             20                  25                  30

CTG AGC GAA GCT TGT GTC TCA GTG ACC AGC TCC ATC TTG AGC TCC ATG        144
Leu Ser Glu Ala Cys Val Ser Val Thr Ser Ser Ile Leu Ser Ser Met
         35                  40                  45

GAC CCC ACA GTG GAC CCC TGC CAT GAC TTC TTC AGC TAC GCC TGT GGG        192
Asp Pro Thr Val Asp Pro Cys His Asp Phe Phe Ser Tyr Ala Cys Gly
     50                  55                  60
```

```
GGC TGG ATC AAG GCC AAC CCA GTC CCT GAT GGC CAC TCA CGC TGG GGG      240
Gly Trp Ile Lys Ala Asn Pro Val Pro Asp Gly His Ser Arg Trp Gly
 65                 70                  75                  80

ACC TTC AGC AAC CTC TGG GAA CAC AAC CAA GCA ATC ATC AAG CAC CTC      288
Thr Phe Ser Asn Leu Trp Glu His Asn Gln Ala Ile Ile Lys His Leu
                85                  90                  95

CTC GAA AAC TCC ACG GCC AGC GTG AGC GAG GCA GAG AGA AAG GCG CAA      336
Leu Glu Asn Ser Thr Ala Ser Val Ser Glu Ala Glu Arg Lys Ala Gln
            100                 105                 110

GTA TAC TAC CGT GCG TGC ATG AAC GAG ACC AGG ATC GAG GAG CTC AGG      384
Val Tyr Tyr Arg Ala Cys Met Asn Glu Thr Arg Ile Glu Glu Leu Arg
        115                 120                 125

GCC AAA CCT CTA ATG GAG TTG ATT GAG AGG CTC GGG GGC TGG AAC ATC      432
Ala Lys Pro Leu Met Glu Leu Ile Glu Arg Leu Gly Gly Trp Asn Ile
    130                 135                 140

ACA GGT CCC TGG GCC AAG GAC AAC TTC CAG GAC ACC CTG CAG GTG GTC      480
Thr Gly Pro Trp Ala Lys Asp Asn Phe Gln Asp Thr Leu Gln Val Val
145                 150                 155                 160

ACC GCC CAC TAC CGC ACC TCA CCC TTC TTC TCT GTC TAT GTC AGT GCC      528
Thr Ala His Tyr Arg Thr Ser Pro Phe Phe Ser Val Tyr Val Ser Ala
                165                 170                 175

GAT TCC AAG AAC TCC AAC AGC AAC GTG ATC CAG GTG GAC CAG TCT GGC      576
Asp Ser Lys Asn Ser Asn Ser Asn Val Ile Gln Val Asp Gln Ser Gly
            180                 185                 190

CTG GGC TTG CCC TCG AGA GAC TAT TAC CTG AAC AAA ACT GAA AAC GAG      624
Leu Gly Leu Pro Ser Arg Asp Tyr Tyr Leu Asn Lys Thr Glu Asn Glu
        195                 200                 205

AAG GTG CTG ACC GGA TAT CTG AAC TAC ATG GTC CAG CTG GGG AAG CTG      672
Lys Val Leu Thr Gly Tyr Leu Asn Tyr Met Val Gln Leu Gly Lys Leu
    210                 215                 220

CTG GGC GGC GGG GAC GAG GAG GCC ATC CGG CCC CAG ATG CAG CAG ATC      720
Leu Gly Gly Gly Asp Glu Glu Ala Ile Arg Pro Gln Met Gln Gln Ile
225                 230                 235                 240

TTG GAC TTT GAG ACG GCA CTG GCC AAC ATC ACC ATC CCA CAG GAG AAG      768
Leu Asp Phe Glu Thr Ala Leu Ala Asn Ile Thr Ile Pro Gln Glu Lys
                245                 250                 255

CGC CGT GAT GAG GAG CTC ATC TAC CAC AAA GTG ACG GCA GCC GAG CTG      816
Arg Arg Asp Glu Glu Leu Ile Tyr His Lys Val Thr Ala Ala Glu Leu
            260                 265                 270
```

```
CAG ACC TTG GCA CCC GCC ATC AAC TGG TTG CCT TTT CTC AAC ACC ATC      864
Gln Thr Leu Ala Pro Ala Ile Asn Trp Leu Pro Phe Leu Asn Thr Ile
        275                 280                 285

TTC TAC CCC GTG GAG ATC AAT GAA TCC GAG CCT ATT GTG GTC TAT GAC      912
Phe Tyr Pro Val Glu Ile Asn Glu Ser Glu Pro Ile Val Val Tyr Asp
    290                 295                 300

AAG GAA TAC CTT GAG CAG ATC TCC ACT CTC ATC AAC ACC ACC GAC AGA      960
Lys Glu Tyr Leu Glu Gln Ile Ser Thr Leu Ile Asn Thr Thr Asp Arg
305                 310                 315                 320

TGC CTG CTC AAC AAC TAC ATG ATC TGG AAC CTG GTG CGG AAA ACA AGC     1008
Cys Leu Leu Asn Asn Tyr Met Ile Trp Asn Leu Val Arg Lys Thr Ser
                325                 330                 335

TCC TTC CTT GAC CAG CGC TTT CAG GAC GCC GAT GAG AAG TTC ATG GAA     105
Ser Phe Leu Asp Gln Arg Phe Gln Asp Ala Asp Glu Lys Phe Met Glu
            340                 345                 350

GTC ATG TAC GGG ACC AAG AAG ACC TGT CTT CCT CGC TGG AAG TTT TGC     1104
Val Met Tyr Gly Thr Lys Lys Thr Cys Leu Pro Arg Trp Lys Phe Cys
        355                 360                 365

GTG AGT GAC ACA GAA AAC AAC CTG GGC TTT GCG TTG GGC CCC ATG TTT     1152
Val Ser Asp Thr Glu Asn Asn Leu Gly Phe Ala Leu Gly Pro Met Phe
    370                 375                 380

GTC AAA GCA ACC TTC GCC GAG GAC AGC AAG AGC ATA GCC ACC GAG ATC     1200
Val Lys Ala Thr Phe Ala Glu Asp Ser Lys Ser Ile Ala Thr Glu Ile
385                 390                 395                 400

ATC CTG GAG ATT AAG AAG GCA TTT GAG GAA AGC CTG AGC ACC CTG AAG     1248
Ile Leu Glu Ile Lys Lys Ala Phe Glu Glu Ser Leu Ser Thr Leu Lys
                405                 410                 415

TGG ATG GAT GAG GAA ACC CGA AAA TCA GCC AAG GAA AAG GCC GAT GCC     1296
Trp Met Asp Glu Glu Thr Arg Lys Ser Ala Lys Glu Lys Ala Asp Ala
            420                 425                 430

ATC TAC AAC ATG ATA GGA TAC CCC AAC TTC ATC ATG GAT CCC AAG GAG     1344
Ile Tyr Asn Met Ile Gly Tyr Pro Asn Phe Ile Met Asp Pro Lys Glu
        435                 440                 445

CTG GAC AAA GTG TTT AAT GAC TAC ACT GCA GTT CCA GAC CTC TAC TTT     1392
Leu Asp Lys Val Phe Asn Asp Tyr Thr Ala Val Pro Asp Leu Tyr Phe
    450                 455                 460

GAA AAT GCC ATG CGG TTT TTC AAC TTC TCA TGG AGG GTC ACT GCC GAT     1440
Glu Asn Ala Met Arg Phe Phe Asn Phe Ser Trp Arg Val Thr Ala Asp
465                 470                 475                 480
```

```
CAG CTC AGG AAA GCC CCC AAC AGA GAT CAG TGG AGC ATG ACC CCG CCC      1488
Gln Leu Arg Lys Ala Pro Asn Arg Asp Gln Trp Ser Met Thr Pro Pro
                485                 490                 495

ATG GTG AAC GCC TAC TAC TCG CCC ACC AAG AAT GAG ATT GTG TTT CCG      1536
Met Val Asn Ala Tyr Tyr Ser Pro Thr Lys Asn Glu Ile Val Phe Pro
            500                 505                 510

GCC GGG ATC CTG CAG GCA CCA TTC TAC ACA CGC TCC TCA CCC AAG GCC      1584
Ala Gly Ile Leu Gln Ala Pro Phe Tyr Thr Arg Ser Ser Pro Lys Ala
        515                 520                 525

TTA AAC TTT GGT GGC ATA GGT GTC GTC GTG GGC CAT GAG CTG ACT CAT      1632
Leu Asn Phe Gly Gly Ile Gly Val Val Val Gly His Glu Leu Thr His
    530                 535                 540

GCT TTT GAT GAT CAA GGA CGG GAG TAT GAC AAG GAC GGG AAC CTC CGG      1680
Ala Phe Asp Asp Gln Gly Arg Glu Tyr Asp Lys Asp Gly Asn Leu Arg
545                 550                 555                 560

CCA TGG TGG AAG AAC TCA TCC GTG GAG GCC TTC AAG CGT CAG ACC GAG      1728
Pro Trp Trp Lys Asn Ser Ser Val Glu Ala Phe Lys Arg Gln Thr Glu
                565                 570                 575

TGC ATG GTA GAG CAG TAC AGC AAC TAC AGC GTG AAC GGG GAG CCG GTG      1776
Cys Met Val Glu Gln Tyr Ser Asn Tyr Ser Val Asn Gly Glu Pro Val
            580                 585                 590

AAC GGG CGG CAC ACC CTG GGG GAG AAC ATC GCC GAC AAC GGG GGT CTC      1824
Asn Gly Arg His Thr Leu Gly Glu Asn Ile Ala Asp Asn Gly Gly Leu
        595                 600                 605

AAG GCG GCC TAT CGG GCT TAC CAG AAC TGG GTG AAG AAG AAC GGG GCT      1872
Lys Ala Ala Tyr Arg Ala Tyr Gln Asn Trp Val Lys Lys Asn Gly Ala
    610                 615                 620

GAG CAC TCG CTC CCC ACC CTG GGC CTC ACC AAT AAC CAG CTC TTC TTC      1920
Glu His Ser Leu Pro Thr Leu Gly Leu Thr Asn Asn Gln Leu Phe Phe
625                 630                 635                 640

CTG GGC TTT GCA CAG GTC TGG TGC TCC GTC CGC ACA CCT GAG AGC TCC      1968
Leu Gly Phe Ala Gln Val Trp Cys Ser Val Arg Thr Pro Glu Ser Ser
                645                 650                 655

CAC GAA GGC CTC ATC ACC GAT CCC CAC AGC CCC TCT CGC TTC CGG GTC      2016
His Glu Gly Leu Ile Thr Asp Pro His Ser Pro Ser Arg Phe Arg Val
            660                 665                 670

ATC GGC TCC CTC TCC AAT TCC AAG GAG TTC TCA GAA CAC TTC CGC TGC      2064
Ile Gly Ser Leu Ser Asn Ser Lys Glu Phe Ser Glu His Phe Arg Cys
        675                 680                 685
```

```
CCA CCT GGC TCA CCC ATG AAC CCG CCT CAC AAG TGC GAA GTC TGG      2109
Pro Pro Gly Ser Pro Met Asn Pro Pro His Lys Cys Glu Val Trp
    690                 695                 700

TAAGGACGAA GCGGAGAGAG CCAAGACGGA GGAGGGGAAG GGGCTGAGGA CGAGACCCCC   2169

ATCCAGCCTC CAGGGCATTG CTCAGCCCGC TTGGCCACCC GGGGCCCTGC TTCCTCACAC   2229

TGGCGGGTTT TCAGCCGGAA CCGAGCCCAT GGTGTTGGCT CTCAACGTGA CCCGCAGTCT   2289

GATCCCCTGT GAAGAGCCGG ACATCCCAGG CACACGTGTG CGCCACCTTC AGCAGGCATT   2349

CGGGTGCTGG GCTGGTGGCT CATCAGGCCT GGGCCCCACA CTGACAAGCG CCAGATACGC   2409

CACAAATACC ACTGTGTCAA ATGCTTTCAA GATATATTTT TGGGGAAACT ATTTTTTAAA   2469

CACTGTGGAA TACACTGGAA ATCTTCAGGG AAAAACACAT TTAAACACTT TTTTTTTTAA   2529

GCCC                                                                2533
```

(2) INFORMATION ZU SEQ ID NO: 25:

(i) SEQUENZ CHARAKTERISTIKA:

(A) LÄNGE: 703 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 25:

```
Arg Leu Val Val Leu Val Val Leu Leu Ala Ala Gly Leu Val Ala Cys
1               5                   10                  15

Leu Ala Ala Leu Gly Ile Gln Tyr Gln Thr Arg Ser Pro Ser Val Cys
            20                  25                  30

Leu Ser Glu Ala Cys Val Ser Val Thr Ser Ser Ile Leu Ser Ser Met
        35                  40                  45

Asp Pro Thr Val Asp Pro Cys His Asp Phe Phe Ser Tyr Ala Cys Gly
    50                  55                  60

Gly Trp Ile Lys Ala Asn Pro Val Pro Asp Gly His Ser Arg Trp Gly
65                  70                  75                  80

Thr Phe Ser Asn Leu Trp Glu His Asn Gln Ala Ile Ile Lys His Leu
                85                  90                  95

Leu Glu Asn Ser Thr Ala Ser Val Ser Glu Ala Glu Arg Lys Ala Gln
            100                 105                 110

Val Tyr Tyr Arg Ala Cys Met Asn Glu Thr Arg Ile Glu Glu Leu Arg
        115                 120                 125
```

Ala Lys Pro Leu Met Glu Leu Ile Glu Arg Leu Gly Gly Trp Asn Ile
130 135 140

Thr Gly Pro Trp Ala Lys Asp Asn Phe Gln Asp Thr Leu Gln Val Val
145 150 155 160

Thr Ala His Tyr Arg Thr Ser Pro Phe Phe Ser Val Tyr Val Ser Ala
165 170 175

Asp Ser Lys Asn Ser Asn Ser Asn Val Ile Gln Val Asp Gln Ser Gly
180 185 190

Leu Gly Leu Pro Ser Arg Asp Tyr Tyr Leu Asn Lys Thr Glu Asn Glu
195 200 205

Lys Val Leu Thr Gly Tyr Leu Asn Tyr Met Val Gln Leu Gly Lys Leu
210 215 220

Leu Gly Gly Gly Asp Glu Glu Ala Ile Arg Pro Gln Met Gln Gln Ile
225 230 235 240

Leu Asp Phe Glu Thr Ala Leu Ala Asn Ile Thr Ile Pro Gln Glu Lys
245 250 255

Arg Arg Asp Glu Glu Leu Ile Tyr His Lys Val Thr Ala Ala Glu Leu
260 265 270

Gln Thr Leu Ala Pro Ala Ile Asn Trp Leu Pro Phe Leu Asn Thr Ile
275 280 285

Phe Tyr Pro Val Glu Ile Asn Glu Ser Glu Pro Ile Val Val Tyr Asp
290 295 300

Lys Glu Tyr Leu Glu Gln Ile Ser Thr Leu Ile Asn Thr Thr Asp Arg
305 310 315 320

Cys Leu Leu Asn Asn Tyr Met Ile Trp Asn Leu Val Arg Lys Thr Ser
325 330 335

Ser Phe Leu Asp Gln Arg Phe Gln Asp Ala Asp Glu Lys Phe Met Glu
340 345 350

Val Met Tyr Gly Thr Lys Lys Thr Cys Leu Pro Arg Trp Lys Phe Cys
355 360 365

Val Ser Asp Thr Glu Asn Asn Leu Gly Phe Ala Leu Gly Pro Met Phe
370 375 380

Val Lys Ala Thr Phe Ala Glu Asp Ser Lys Ser Ile Ala Thr Glu Ile
385 390 395 400

Ile Leu Glu Ile Lys Lys Ala Phe Glu Glu Ser Leu Ser Thr Leu Lys
405 410 415

```
Trp Met Asp Glu Glu Thr Arg Lys Ser Ala Lys Glu Lys Ala Asp Ala
            420                 425                 430

Ile Tyr Asn Met Ile Gly Tyr Pro Asn Phe Ile Met Asp Pro Lys Glu
        435                 440                 445

Leu Asp Lys Val Phe Asn Asp Tyr Thr Ala Val Pro Asp Leu Tyr Phe
    450                 455                 460

Glu Asn Ala Met Arg Phe Phe Asn Phe Ser Trp Arg Val Thr Ala Asp
465                 470                 475                 480

Gln Leu Arg Lys Ala Pro Asn Arg Asp Gln Trp Ser Met Thr Pro Pro
                485                 490                 495

Met Val Asn Ala Tyr Tyr Ser Pro Thr Lys Asn Glu Ile Val Phe Pro
            500                 505                 510

Ala Gly Ile Leu Gln Ala Pro Phe Tyr Thr Arg Ser Ser Pro Lys Ala
        515                 520                 525

Leu Asn Phe Gly Gly Ile Gly Val Val Val Gly His Glu Leu Thr His
    530                 535                 540

Ala Phe Asp Asp Gln Gly Arg Glu Tyr Asp Lys Asp Gly Asn Leu Arg
545                 550                 555                 560

Pro Trp Trp Lys Asn Ser Ser Val Glu Ala Phe Lys Arg Gln Thr Glu
                565                 570                 575

Cys Met Val Glu Gln Tyr Ser Asn Tyr Ser Val Asn Gly Glu Pro Val
            580                 585                 590

Asn Gly Arg His Thr Leu Gly Glu Asn Ile Ala Asp Asn Gly Gly Leu
        595                 600                 605

Lys Ala Ala Tyr Arg Ala Tyr Gln Asn Trp Val Lys Lys Asn Gly Ala
    610                 615                 620

Glu His Ser Leu Pro Thr Leu Gly Leu Thr Asn Asn Gln Leu Phe Phe
625                 630                 635                 640

Leu Gly Phe Ala Gln Val Trp Cys Ser Val Arg Thr Pro Glu Ser Ser
                645                 650                 655

His Glu Gly Leu Ile Thr Asp Pro His Ser Pro Ser Arg Phe Arg Val
            660                 665                 670

Ile Gly Ser Leu Ser Asn Ser Lys Glu Phe Ser Glu His Phe Arg Cys
        675                 680                 685

Pro Pro Gly Ser Pro Met Asn Pro Pro His Lys Cys Glu Val Trp
    690                 695                 700
```

(2) INFORMATION ZU SEQ ID NO: 26:

(i) SEQUENZ CHARAKTERISTIKA:

(A) LÄNGE: 24 Basenpaare
(B) ART: Nukleinsäure
(C) STRANGFORM: Einzel
(D) TOPOLOGIE: linear

(ii) ART DES MOLEKÜLS: DNS (genomisch)
(iii) HYPOTHETISCH: NEIN
(iii) ANTISENSE: JA
(xi) SEQUENZBESCHREIBUNG: SEQ ID NO: 26:

GGTGCTTGAT GATGGCTTGG TTGT 24

(2) INFORMATION ZU SEQ ID NO: 27:

(i) SEQUENZ CHARAKTERISTIKA:

(A) LÄNGE: 24 Basenpaare
(B) ART: Nukleinsäure
(C) STRANGFORM: Einzel
(D) TOPOLOGIE: linear

(ii) ART DES MOLEKÜLS: DNS (genomisch)
(iii) HYPOTHETISCH: NEIN
(iii) ANTISENSE: JA
(xi) SEQUENZBESCHREIBUNG: SEQ ID NO: 27:

AGATGGAGCT GGTCACCGAG ATGC 24

(2) INFORMATION ZU SEQ ID NO: 28:

(i) SEQUENZ CHARAKTERISTIKA:

(A) LÄNGE: 324 Basenpaare
(B) ART: Nukleinsäure
(C) STRANGFORM: Einzel
(D) TOPOLOGIE: linear

(ii) ART DES MOLEKÜLS: cDNS zu mRNS
(iii) HYPOTHETISCH: NEIN
(vi) URSPRÜNLICHE HERKUNFT:

(A) ORGANISMUS: Bos taurus
(F) GEWEBETYP: Lunge

(xi) SEQUENZBESCHREIBUNG: SEQ ID NO: 28:

GGGAGCGCGG CGCGGGGCCG GAGCGGAGCG CGCGAGCGAT GATGTCTACC TACAAGCGGC 60

```
CCACGCTGGA CGAGGAGGAC CTGGTGGACT CGCTGTCCGA GAGCGACGTG TACCCCAACC    120
ACCTGCAGGT GAACTTCCGA GGCCCCCGGA ACGGCCAGAG ATGCTGGGCC GCCAGGACCC    180
CGGTGGAGAA GCGGCTGGTG GTGCTGGTGG CGCTCCTGGC GGCGGCATTG GTGGCCTGTT    240
TGGCAGTACT GGGCATCCAA TACCAGACAA GAACGCCCTC GGTGTGCCTA AGTGAGGCCT    300
GCATCTCGGT GACCAGCTCC ATCT                                           324
```

(2) INFORMATION ZU SEQ ID NO: 29:

(i) SEQUENZ CHARAKTERISTIKA:

(A) LÄNGE: 2314 Basenpaare
(B) ART: Nukleinsäure
(C) STRANGFORM: Einzel
(D) TOPOLOGIE: linear

(ii) ART DES MOLEKÜLS: cDNS zu mRNS
(vi) URSPRÜNLICHE HERKUNFT:

(A) ORGANISMUS: Bos taurus
(F) GEWEBETYP: Lunge

(ix) MERKMALE:

(A) NAME/SCHLÜSSEL: CDS
(B) LAGE: 39..2301

(xi) SEQUENZBESCHREIBUNG: SEQ ID NO: 29:

```
GGGAGCGCGG CGCGGGGCCG GAGCGGAGCG CGCGAGCG ATG ATG TCT ACC TAC          53
                                         Met Met Ser Thr Tyr
                                          1               5

AAG CGG CCC ACG CTG GAC GAG GAG GAC CTG GTG GAC TCG CTG TCC GAG       101
Lys Arg Pro Thr Leu Asp Glu Glu Asp Leu Val Asp Ser Leu Ser Glu
                     10                  15                  20

AGC GAC GTG TAC CCC AAC CAC CTG CAG GTG AAC TTC CGA GGC CCC CGG       149
Ser Asp Val Tyr Pro Asn His Leu Gln Val Asn Phe Arg Gly Pro Arg
                 25                  30                  35

AAC GGC CAG AGA TGC TGG GCC GCC AGG ACC CCG GTG GAG AAG CGG CTG       197
Asn Gly Gln Arg Cys Trp Ala Ala Arg Thr Pro Val Glu Lys Arg Leu
             40                  45                  50

GTG GTG CTG GTG GCG CTC CTG GCG GCG GCA TTG GTG GCC TGT TTG GCA       245
Val Val Leu Val Ala Leu Leu Ala Ala Ala Leu Val Ala Cys Leu Ala
         55                  60                  65

GTA CTG GGC ATC CAA TAC CAG ACA AGA ACG CCC TCG GTG TGC CTA AGT       293
Val Leu Gly Ile Gln Tyr Gln Thr Arg Thr Pro Ser Val Cys Leu Ser
 70                  75                  80                  85
```

```
GAG GCC TGC ATC TCG GTG ACC AGC TCC ATC TTG AGT TCC ATG GAC CCC      341
Glu Ala Cys Ile Ser Val Thr Ser Ser Ile Leu Ser Ser Met Asp Pro
                 90                  95                 100

ACG GTG GAC CCC TGC CAG GAC TTC TTC ACC TAT GCC TGT GGC GGC TGG      389
Thr Val Asp Pro Cys Gln Asp Phe Phe Thr Tyr Ala Cys Gly Gly Trp
            105                 110                 115

ATC AAA GCC AAC CCC GTG CCG GAT GGC CAC TCG CGC TGG GGG ACC TTC      437
Ile Lys Ala Asn Pro Val Pro Asp Gly His Ser Arg Trp Gly Thr Phe
        120                 125                 130

AGC AAC CTC TGG GAA CAC AAC CAA GCC ATC ATC AAG CAC CTC CTT GAA      485
Ser Asn Leu Trp Glu His Asn Gln Ala Ile Ile Lys His Leu Leu Glu
    135                 140                 145

AAC TCC ACG GCC AGC GTG AGC GAG GCA GAG AGG AAG GAC CAG GAG TAC      533
Asn Ser Thr Ala Ser Val Ser Glu Ala Glu Arg Lys Asp Gln Glu Tyr
150                 155                 160                 165

TAC CGA GCC TGC ATG AAC GAA ACC AGG ATT GAG GAG CTC AAG GCC AAA      581
Tyr Arg Ala Cys Met Asn Glu Thr Arg Ile Glu Glu Leu Lys Ala Lys
                170                 175                 180

CCC CTG ATG GAG CTC ATT GAG AAG CTC GGC GGC TGG AAC ATC ACG GGG      629
Pro Leu Met Glu Leu Ile Glu Lys Leu Gly Gly Trp Asn Ile Thr Gly
            185                 190                 195

CCC TGG GAC AAG GAC AAC TTC CAG GAC ACC CTG CAG GTG GTC ACA TCC      677
Pro Trp Asp Lys Asp Asn Phe Gln Asp Thr Leu Gln Val Val Thr Ser
        200                 205                 210

CAC TAC CAC ACC TCC CCC TTC TTC TCC GTC TAC GTC AGT GCC GAC TCC      725
His Tyr His Thr Ser Pro Phe Phe Ser Val Tyr Val Ser Ala Asp Ser
    215                 220                 225

AAG AAT TCC AAC AGC AAC GTG ATC CAA GTG GAC CAG TCT GGC CTG GGC      773
Lys Asn Ser Asn Ser Asn Val Ile Gln Val Asp Gln Ser Gly Leu Gly
230                 235                 240                 245

TTA CCC TCA AGA GAT TAT TAC CTG AAC AAA ACC GAG AAT GAG AAG GTG      821
Leu Pro Ser Arg Asp Tyr Tyr Leu Asn Lys Thr Glu Asn Glu Lys Val
                250                 255                 260

CTG ACG GGA TAC CTG AAC TAC ATG GTC CAG CTG GGG AAG CTG CTG GGA      869
Leu Thr Gly Tyr Leu Asn Tyr Met Val Gln Leu Gly Lys Leu Leu Gly
            265                 270                 275

GGA GGG GCC GAG GAC ACC ATC CGG CCC CAG ATG CAG CAG ATC CTG GAC      917
Gly Gly Ala Glu Asp Thr Ile Arg Pro Gln Met Gln Gln Ile Leu Asp
        280                 285                 290
```

```
TTT GAG ACG GCG CTG GCC AAC ATC ACC ATC CCC CAG GAG AAG CGC CGG        965
Phe Glu Thr Ala Leu Ala Asn Ile Thr Ile Pro Gln Glu Lys Arg Arg
    295                 300                 305

GAC GAG GAA CTC ATC TAC CAC AAA GTG ACG GCG GCT GAG TTG CAG ACC       1013
Asp Glu Glu Leu Ile Tyr His Lys Val Thr Ala Ala Glu Leu Gln Thr
310                 315                 320                 325

TTG GCG CCC GCC ATC AAC TGG CTG CCC TTC CTC AAC ACC ATC TTC TAC       1061
Leu Ala Pro Ala Ile Asn Trp Leu Pro Phe Leu Asn Thr Ile Phe Tyr
                330                 335                 340

CCC GTG GAG ATC AAT GAA TCA GAG CCT ATT GTC ATC TAC GAC AAA GAA       1109
Pro Val Glu Ile Asn Glu Ser Glu Pro Ile Val Ile Tyr Asp Lys Glu
            345                 350                 355

TAC CTG AGC AAG GTC TCC ACC CTC ATC AAC AGC ACA GAC AAA TGC CTG       1157
Tyr Leu Ser Lys Val Ser Thr Leu Ile Asn Ser Thr Asp Lys Cys Leu
        360                 365                 370

CTG AAC AAC TAC ATG ATC TGG AAC CTG GTA CGG AAG ACG AGC TCC TTC       1205
Leu Asn Asn Tyr Met Ile Trp Asn Leu Val Arg Lys Thr Ser Ser Phe
    375                 380                 385

CTC GAT CAG CGC TTC CAG GAC GCC GAC GAG AAG TTC ATG GAA GTC ATG       1253
Leu Asp Gln Arg Phe Gln Asp Ala Asp Glu Lys Phe Met Glu Val Met
390                 395                 400                 405

TAT GGG ACC AAG AAG ACG TGT CTT CCC CGC TGG AAG TTT TGT GTG AGT       1301
Tyr Gly Thr Lys Lys Thr Cys Leu Pro Arg Trp Lys Phe Cys Val Ser
                410                 415                 420

GAT ACA GAG AAC ACC TTG GGC TTC GCC CTG GGC CCC ATG TTC GTC AAA       1349
Asp Thr Glu Asn Thr Leu Gly Phe Ala Leu Gly Pro Met Phe Val Lys
            425                 430                 435

GCG ACC TTC GCT GAG GAC AGC AAG AAC ATA GCC AGC GAG ATC ATC CTG       1397
Ala Thr Phe Ala Glu Asp Ser Lys Asn Ile Ala Ser Glu Ile Ile Leu
        440                 445                 450

GAG ATC AAG AAG GCG TTT GAA GAG AGC CTG AGC ACC CTG AAG TGG ATG       1445
Glu Ile Lys Lys Ala Phe Glu Glu Ser Leu Ser Thr Leu Lys Trp Met
    455                 460                 465

GAT GAA GAT ACT CGG AAA TCG GCC AAG GAA AAG GCG GAC GCG ATC TAC       1493
Asp Glu Asp Thr Arg Lys Ser Ala Lys Glu Lys Ala Asp Ala Ile Tyr
470                 475                 480                 485

AAC ATG ATA GGC TAC CCC AAC TTT ATC ATG GAC CCC AAG GAG CTG GAC       1541
Asn Met Ile Gly Tyr Pro Asn Phe Ile Met Asp Pro Lys Glu Leu Asp
                490                 495                 500
```

```
AAA GTG TTC AAT GAC TAC ACC GCT GTG CCA GAC CTC TAC TTC GAG AAC     1589
Lys Val Phe Asn Asp Tyr Thr Ala Val Pro Asp Leu Tyr Phe Glu Asn
            505                 510                 515

GCC ATG CGG TTT TTC AAC TTC TCC TGG AGG GTC ACT GCC GAC CAG CTC     1637
Ala Met Arg Phe Phe Asn Phe Ser Trp Arg Val Thr Ala Asp Gln Leu
        520                 525                 530

CGG AAA GCG CCC AAC AGA GAT CAG TGG AGC ATG ACC CCG CCC ATG GTG     1685
Arg Lys Ala Pro Asn Arg Asp Gln Trp Ser Met Thr Pro Pro Met Val
    535                 540                 545

AAC GCC TAC TAC TCG CCC ACC AAG AAC GAG ATC GTG TTT CCG GCC GGA     1733
Asn Ala Tyr Tyr Ser Pro Thr Lys Asn Glu Ile Val Phe Pro Ala Gly
550                 555                 560                 565

ATC CTG CAG GCG CCA TTC TAC ACC CGC TCT TCA CCC AAT GCC TTA AAC     1781
Ile Leu Gln Ala Pro Phe Tyr Thr Arg Ser Ser Pro Asn Ala Leu Asn
                570                 575                 580

TTC GGC GGC ATC GGC GTC GTC GTG GGC CAC GAG CTG ACT CAT GCT TTT     1829
Phe Gly Gly Ile Gly Val Val Val Gly His Glu Leu Thr His Ala Phe
            585                 590                 595

GAT GAT CAA GGC CGA GAG TAC GAC AAG GAT GGG AAC CTC CGG CCC TGG     1877
Asp Asp Gln Gly Arg Glu Tyr Asp Lys Asp Gly Asn Leu Arg Pro Trp
        600                 605                 610

TGG AAG AAC TCG TCC GTG GAG GCG TTC AAG CAG CAG ACC GCG TGC ATG     1925
Trp Lys Asn Ser Ser Val Glu Ala Phe Lys Gln Gln Thr Ala Cys Met
    615                 620                 625

GTG GAG CAG TAC GGC AAC TAT AGC GTG AAC GGG GAG CCG GTG AAC GGC     1973
Val Glu Gln Tyr Gly Asn Tyr Ser Val Asn Gly Glu Pro Val Asn Gly
630                 635                 640                 645

CGG CAC ACC CTC GGC GAA AAC ATC GCC GAC AAC GGG GGC CTC AAG GCG     2021
Arg His Thr Leu Gly Glu Asn Ile Ala Asp Asn Gly Gly Leu Lys Ala
                650                 655                 660

GCC TAT CGG GCC TAC CAG AAC TGG GTC AAG AAG AAT GGG GCT GAG CAG     2069
Ala Tyr Arg Ala Tyr Gln Asn Trp Val Lys Lys Asn Gly Ala Glu Gln
            665                 670                 675

ACA CTG CCC ACC CTG GGT CTC ACC AAC AAC CAG CTC TTC TTC CTG AGT     2117
Thr Leu Pro Thr Leu Gly Leu Thr Asn Asn Gln Leu Phe Phe Leu Ser
        680                 685                 690

TTT GCA CAG GTC TGG TGT TCC GTC CGC ACC CCC GAG AGT TCG CAC GAA     2165
Phe Ala Gln Val Trp Cys Ser Val Arg Thr Pro Glu Ser Ser His Glu
    695                 700                 705
```

```
GGT CTC ATC ACC GAT CCC CAC AGC CCC TCC CGC TTC CGG GTC ATC GGC       2213
Gly Leu Ile Thr Asp Pro His Ser Pro Ser Arg Phe Arg Val Ile Gly
710                 715                 720                 725

TCC ATC TCC AAC TCC AAG GAG TTC TCG GAA CAC TTC CAC TGC CCG CCC       2261
Ser Ile Ser Asn Ser Lys Glu Phe Ser Glu His Phe His Cys Pro Pro
                730                 735                 740

GGC TCA CCC ATG AAC CCG CAT CAC AAG TGT GAA GTC TGG T GAAGGGCCAG      2311
Gly Ser Pro Met Asn Pro His His Lys Cys Glu Val Trp
            745                 750

GCA                                                                   2314
```

(2) INFORMATION ZU SEQ ID NO: 30:

(i) SEQUENZ CHARAKTERISTIKA:

(A) LÄNGE: 754 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 30:

Met Met Ser Thr Tyr Lys Arg Pro Thr Leu Asp Glu Glu Asp Leu Val
1 5 10 15

Asp Ser Leu Ser Glu Ser Asp Val Tyr Pro Asn His Leu Gln Val Asn
20 25 30

Phe Arg Gly Pro Arg Asn Gly Gln Arg Cys Trp Ala Ala Arg Thr Pro
35 40 45

Val Glu Lys Arg Leu Val Val Leu Val Ala Leu Leu Ala Ala Ala Leu
50 55 60

Val Ala Cys Leu Ala Val Leu Gly Ile Gln Tyr Gln Thr Arg Thr Pro
65 70 75 80

Ser Val Cys Leu Ser Glu Ala Cys Ile Ser Val Thr Ser Ser Ile Leu
85 90 95

Ser Ser Met Asp Pro Thr Val Asp Pro Cys Gln Asp Phe Phe Thr Tyr
100 105 110

Ala Cys Gly Gly Trp Ile Lys Ala Asn Pro Val Pro Asp Gly His Ser
115 120 125

Arg Trp Gly Thr Phe Ser Asn Leu Trp Glu His Asn Gln Ala Ile Ile
130 135 140

Lys His Leu Leu Glu Asn Ser Thr Ala Ser Val Ser Glu Ala Glu Arg
145 150 155 160

```
Lys Asp Gln Glu Tyr Tyr Arg Ala Cys Met Asn Glu Thr Arg Ile Glu
                165                 170                 175

Glu Leu Lys Ala Lys Pro Leu Met Glu Leu Ile Glu Lys Leu Gly Gly
            180                 185                 190

Trp Asn Ile Thr Gly Pro Trp Asp Lys Asp Asn Phe Gln Asp Thr Leu
        195                 200                 205

Gln Val Val Thr Ser His Tyr His Thr Ser Pro Phe Phe Ser Val Tyr
    210                 215                 220

Val Ser Ala Asp Ser Lys Asn Ser Asn Ser Asn Val Ile Gln Val Asp
225                 230                 235                 240

Gln Ser Gly Leu Gly Leu Pro Ser Arg Asp Tyr Tyr Leu Asn Lys Thr
                245                 250                 255

Glu Asn Glu Lys Val Leu Thr Gly Tyr Leu Asn Tyr Met Val Gln Leu
            260                 265                 270

Gly Lys Leu Leu Gly Gly Gly Ala Glu Asp Thr Ile Arg Pro Gln Met
        275                 280                 285

Gln Gln Ile Leu Asp Phe Glu Thr Ala Leu Ala Asn Ile Thr Ile Pro
    290                 295                 300

Gln Glu Lys Arg Arg Asp Glu Glu Leu Ile Tyr His Lys Val Thr Ala
305                 310                 315                 320

Ala Glu Leu Gln Thr Leu Ala Pro Ala Ile Asn Trp Leu Pro Phe Leu
                325                 330                 335

Asn Thr Ile Phe Tyr Pro Val Glu Ile Asn Glu Ser Glu Pro Ile Val
            340                 345                 350

Ile Tyr Asp Lys Glu Tyr Leu Ser Lys Val Ser Thr Leu Ile Asn Ser
        355                 360                 365

Thr Asp Lys Cys Leu Leu Asn Asn Tyr Met Ile Trp Asn Leu Val Arg
    370                 375                 380

Lys Thr Ser Ser Phe Leu Asp Gln Arg Phe Gln Asp Ala Asp Glu Lys
385                 390                 395                 400

Phe Met Glu Val Met Tyr Gly Thr Lys Lys Thr Cys Leu Pro Arg Trp
                405                 410                 415

Lys Phe Cys Val Ser Asp Thr Glu Asn Thr Leu Gly Phe Ala Leu Gly
            420                 425                 430

Pro Met Phe Val Lys Ala Thr Phe Ala Glu Asp Ser Lys Asn Ile Ala
        435                 440                 445
```

Ser Glu Ile Ile Leu Glu Ile Lys Lys Ala Phe Glu Glu Ser Leu Ser
450 455 460

Thr Leu Lys Trp Met Asp Glu Asp Thr Arg Lys Ser Ala Lys Glu Lys
465 470 475 480

Ala Asp Ala Ile Tyr Asn Met Ile Gly Tyr Pro Asn Phe Ile Met Asp
485 490 495

Pro Lys Glu Leu Asp Lys Val Phe Asn Asp Tyr Thr Ala Val Pro Asp
500 505 510

Leu Tyr Phe Glu Asn Ala Met Arg Phe Phe Asn Phe Ser Trp Arg Val
515 520 525

Thr Ala Asp Gln Leu Arg Lys Ala Pro Asn Arg Asp Gln Trp Ser Met
530 535 540

Thr Pro Pro Met Val Asn Ala Tyr Tyr Ser Pro Thr Lys Asn Glu Ile
545 550 555 560

Val Phe Pro Ala Gly Ile Leu Gln Ala Pro Phe Tyr Thr Arg Ser Ser
565 570 575

Pro Asn Ala Leu Asn Phe Gly Gly Ile Gly Val Val Val Gly His Glu
580 585 590

Leu Thr His Ala Phe Asp Asp Gln Gly Arg Glu Tyr Asp Lys Asp Gly
595 600 605

Asn Leu Arg Pro Trp Trp Lys Asn Ser Ser Val Glu Ala Phe Lys Gln
610 615 620

Gln Thr Ala Cys Met Val Glu Gln Tyr Gly Asn Tyr Ser Val Asn Gly
625 630 635 640

Glu Pro Val Asn Gly Arg His Thr Leu Gly Glu Asn Ile Ala Asp Asn
645 650 655

Gly Gly Leu Lys Ala Ala Tyr Arg Ala Tyr Gln Asn Trp Val Lys Lys
660 665 670

Asn Gly Ala Glu Gln Thr Leu Pro Thr Leu Gly Leu Thr Asn Asn Gln
675 680 685

Leu Phe Phe Leu Ser Phe Ala Gln Val Trp Cys Ser Val Arg Thr Pro
690 695 700

Glu Ser Ser His Glu Gly Leu Ile Thr Asp Pro His Ser Pro Ser Arg
705 710 715 720

Phe Arg Val Ile Gly Ser Ile Ser Asn Ser Lys Glu Phe Ser Glu His
725 730 735

Phe His Cys Pro Pro Gly Ser Pro Met Asn Pro His His Lys Cys Glu
740 745 750

Val Trp

(2) INFORMATION ZU SEQ ID NO: 31:

(i) SEQUENZ CHARAKTERISTIKA:

(A) LÄNGE: 53 Basenpaare
(B) ART: Nukleinsäure
(C) STRANGFORM: Einzel
(D) TOPOLOGIE: linear

(ii) ART DES MOLEKÜLS: DNS (genomisch)
(iii) HYPOTHETISCH: NEIN
(iii) ANTISENSE: JA
(xi) SEQUENZBESCHREIBUNG: SEQ ID NO: 31:

GAGAGAGAGA GAGAGAGAGA ACTAGTCTCG AGCCAAGCAG GCCACCAGTC CTG 53

(2) INFORMATION ZU SEQ ID NO: 32:

(i) SEQUENZ CHARAKTERISTIKA:

(A) LÄNGE: 25 Basenpaare
(B) ART: Nukleinsäure
(C) STRANGFORM: Einzel
(D) TOPOLOGIE: linear

(ii) ART DES MOLEKÜLS: DNS (genomisch)
(iii) HYPOTHETISCH: NEIN
(iii) ANTISENSE: JA
(xi) SEQUENZBESCHREIBUNG: SEQ ID NO: 32:

CCTGCCGCCA GAAGTACCAC CAACA 25

(2) INFORMATION ZU SEQ ID NO: 33:

(i) SEQUENZ CHARAKTERISTIKA:

(A) LÄNGE: 222 Basenpaare
(B) ART: Nukleinsäure
(C) STRANGFORM: Einzel
(D) TOPOLOGIE: linear

(ii) ART DES MOLEKÜLS: cDNS zu mRNS
(iii) HYPOTHETISCH: NEIN
(vi) URSPRÜNLICHE HERKUNFT:

(A) ORGANISMUS: Homo sapiens

(F) GEWEBETYP: Plazenta

(ix) MERKMALE:

(A) NAME/SCHLÜSSEL: CDS
(B) LAGE: 38..222

(xi) SEQUENZBESCHREIBUNG: SEQ ID NO: 33:

```
CGCCCCCCCG GTGTCCGCCC TGCTGTCGGC GCTGGGG ATG TCG ACG TAC AAG CGG        55
                                        Met Ser Thr Tyr Lys Arg
                                          1               5

GCC ACG CTG GAC GAG GAG GAC CTG GTG GAC TCG CTC TCC GAG GGC GAC        103
Ala Thr Leu Asp Glu Glu Asp Leu Val Asp Ser Leu Ser Glu Gly Asp
            10                  15                  20

GCA TAC CCC AAC GGC CTG CAG GTG AAC TTC CAC AGC CCC CGG AGT GGC        151
Ala Tyr Pro Asn Gly Leu Gln Val Asn Phe His Ser Pro Arg Ser Gly
        25                  30                  35

CAG AGG TGC TGG GCT GCA CGG ACC CAG GTG GAG AAG CGG CTG GTG GTG        199
Gln Arg Cys Trp Ala Ala Arg Thr Gln Val Glu Lys Arg Leu Val Val
    40                  45                  50

TTG GTG GTA CTT CTG GCG GCA GG                                         222
Leu Val Val Leu Leu Ala Ala
 55                  60
```

(2) INFORMATION ZU SEQ ID NO: 34:

(i) SEQUENZ CHARAKTERISTIKA:

(A) LÄNGE: 61 Aminosäuren
(B) ART: Aminosäure
(D) TOPOLOGIE: linear

(ii) ART DES MOLEKÜLS: Protein
(xi) SEQUENZBESCHREIDUNG: SEQ ID NO: 34:

```
Met Ser Thr Tyr Lys Arg Ala Thr Leu Asp Glu Glu Asp Leu Val Asp
  1               5                  10                  15

Ser Leu Ser Glu Gly Asp Ala Tyr Pro Asn Gly Leu Gln Val Asn Phe
            20                  25                  30

His Ser Pro Arg Ser Gly Gln Arg Cys Trp Ala Ala Arg Thr Gln Val
        35                  40                  45

Glu Lys Arg Leu Val Val Leu Val Val Leu Leu Ala Ala
    50                  55                  60
```

(2) INFORMATION ZU SEQ ID NO: 35:

(i) SEQUENZ CHARAKTERISTIKA:

(A) LÄNGE: 2720 Basenpaare
(B) ART: Nukleinsäure
(C) STRANGFORM: Einzel
(D) TOPOLOGIE: linear

(ii) ART DES MOLEKÜLS: cDNS zu mRNS
(iii) HYPOTHETISCH: NEIN
(vi) URSPRÜNLICHE HERKUNFT:

(A) ORGANISMUS: Homo sapiens
(F) GEWEBETYP: Plazenta

(ix) MERKMALE:

(A) NAME/SCHLL1SSEL: CDS
(B) LAGE: 38..2297

(xi) SEQUENZBESCHREIBUNG: SEQ ID NO: 35:

```
CGCCCCCCCG GTGTCCGCCC TGCTGTCGGC GCTGGGG ATG TCG ACG TAC AAG CGG      55
                                         Met Ser Thr Tyr Lys Arg
                                          1               5

GCC ACG CTG GAC GAG GAG GAC CTG GTG GAC TCG CTC TCC GAG GGC GAC      103
Ala Thr Leu Asp Glu Glu Asp Leu Val Asp Ser Leu Ser Glu Gly Asp
            10                  15                  20

GCA TAC CCC AAC GGC CTG CAG GTG AAC TTC CAC AGC CCC CGG AGT GGC      151
Ala Tyr Pro Asn Gly Leu Gln Val Asn Phe His Ser Pro Arg Ser Gly
        25                  30                  35

CAG AGG TGC TGG GCT GCA CGG ACC CAG GTG GAG AAG CGG CTG GTG GTG      199
Gln Arg Cys Trp Ala Ala Arg Thr Gln Val Glu Lys Arg Leu Val Val
    40                  45                  50

TTG GTG GTA CTT CTG GCG GCA GGA CTG GTG GCC TGC TTG GCA GCA CTG      247
Leu Val Val Leu Leu Ala Ala Gly Leu Val Ala Cys Leu Ala Ala Leu
55                  60                  65                  70

GGC ATC CAG TAC CAG ACA AGA TCC CCC TCT GTG TGC CTG AGC GAA GCT      295
Gly Ile Gln Tyr Gln Thr Arg Ser Pro Ser Val Cys Leu Ser Glu Ala
                75                  80                  85

TGT GTC TCA GTG ACC AGC TCC ATC TTG AGC TCC ATG GAC CCC ACA GTG      343
Cys Val Ser Val Thr Ser Ser Ile Leu Ser Ser Met Asp Pro Thr Val
            90                  95                  100

GAC CCC TGC CAT GAC TTC TTC AGC TAC GCC TGT GGG GGC TGG ATC AAG      391
Asp Pro Cys His Asp Phe Phe Ser Tyr Ala Cys Gly Gly Trp Ile Lys
        105                 110                 115
```

```
GCC AAC CCA GTC CCT GAT GGC CAC TCA CGC TGG GGG ACC TTC AGC AAC      439
Ala Asn Pro Val Pro Asp Gly His Ser Arg Trp Gly Thr Phe Ser Asn
    120                 125                 130

CTC TGG GAA CAC AAC CAA GCA ATC ATC AAG CAC CTC CTC GAA AAC TCC      487
Leu Trp Glu His Asn Gln Ala Ile Ile Lys His Leu Leu Glu Asn Ser
135                 140                 145                 150

ACG GCC AGC GTG AGC GAG GCA GAG AGA AAG GCG CAA GTA TAC TAC CGT      535
Thr Ala Ser Val Ser Glu Ala Glu Arg Lys Ala Gln Val Tyr Tyr Arg
                155                 160                 165

GCG TGC ATG AAC GAG ACC AGG ATC GAG GAG CTC AGG GCC AAA CCT CTA      583
Ala Cys Met Asn Glu Thr Arg Ile Glu Glu Leu Arg Ala Lys Pro Leu
            170                 175                 180

ATG GAG TTG ATT GAG AGG CTC GGG GGC TGG AAC ATC ACA GGT CCC TGG      631
Met Glu Leu Ile Glu Arg Leu Gly Gly Trp Asn Ile Thr Gly Pro Trp
        185                 190                 195

GCC AAG GAC AAC TTC CAG GAC ACC CTG CAG GTG GTC ACC GCC CAC TAC      679
Ala Lys Asp Asn Phe Gln Asp Thr Leu Gln Val Val Thr Ala His Tyr
    200                 205                 210

CGC ACC TCA CCC TTC TTC TCT GTC TAT GTC AGT GCC GAT TCC AAG AAC      727
Arg Thr Ser Pro Phe Phe Ser Val Tyr Val Ser Ala Asp Ser Lys Asn
215                 220                 225                 230

TCC AAC AGC AAC GTG ATC CAG GTG GAC CAG TCT GGC CTG GGC TTG CCC      775
Ser Asn Ser Asn Val Ile Gln Val Asp Gln Ser Gly Leu Gly Leu Pro
                235                 240                 245

TCG AGA GAC TAT TAC CTG AAC AAA ACT GAA AAC GAG AAG GTG CTG ACC      823
Ser Arg Asp Tyr Tyr Leu Asn Lys Thr Glu Asn Glu Lys Val Leu Thr
            250                 255                 260

GGA TAT CTG AAC TAC ATG GTC CAG CTG GGG AAG CTG CTG GGC GGC GGG      871
Gly Tyr Leu Asn Tyr Met Val Gln Leu Gly Lys Leu Leu Gly Gly Gly
        265                 270                 275

GAC GAG GAG GCC ATC CGG CCC CAG ATG CAG CAG ATC TTG GAC TTT GAG      919
Asp Glu Glu Ala Ile Arg Pro Gln Met Gln Gln Ile Leu Asp Phe Glu
    280                 285                 290

ACG GCA CTG GCC AAC ATC ACC ATC CCA CAG GAG AAG CGC CGT GAT GAG      967
Thr Ala Leu Ala Asn Ile Thr Ile Pro Gln Glu Lys Arg Arg Asp Glu
295                 300                 305                 310

GAG CTC ATC TAC CAC AAA GTG ACG GCA GCC GAG CTG CAG ACC TTG GCA     1015
Glu Leu Ile Tyr His Lys Val Thr Ala Ala Glu Leu Gln Thr Leu Ala
                315                 320                 325
```

```
CCC GCC ATC AAC TGG TTG CCT TTT CTC AAC ACC ATC TTC TAC CCC GTG     1063
Pro Ala Ile Asn Trp Leu Pro Phe Leu Asn Thr Ile Phe Tyr Pro Val
            330                 335                 340

GAG ATC AAT GAA TCC GAG CCT ATT GTG GTC TAT GAC AAG GAA TAC CTT     1111
Glu Ile Asn Glu Ser Glu Pro Ile Val Val Tyr Asp Lys Glu Tyr Leu
        345                 350                 355

GAG CAG ATC TCC ACT CTC ATC AAC ACC ACC GAC AGA TGC CTG CTC AAC     1159
Glu Gln Ile Ser Thr Leu Ile Asn Thr Thr Asp Arg Cys Leu Leu Asn
    360                 365                 370

AAC TAC ATG ATC TGG AAC CTG GTG CGG AAA ACA AGC TCC TTC CTT GAC     1207
Asn Tyr Met Ile Trp Asn Leu Val Arg Lys Thr Ser Ser Phe Leu Asp
375                 380                 385                 390

CAG CGC TTT CAG GAC GCC GAT GAG AAG TTC ATG GAA GTC ATG TAC GGG     1255
Gln Arg Phe Gln Asp Ala Asp Glu Lys Phe Met Glu Val Met Tyr Gly
                395                 400                 405

ACC AAG AAG ACC TGT CTT CCT CGC TGG AAG TTT TGC GTG AGT GAC ACA     1303
Thr Lys Lys Thr Cys Leu Pro Arg Trp Lys Phe Cys Val Ser Asp Thr
            410                 415                 420

GAA AAC AAC CTG GGC TTT GCG TTG GGC CCC ATG TTT GTC AAA GCA ACC     1351
Glu Asn Asn Leu Gly Phe Ala Leu Gly Pro Met Phe Val Lys Ala Thr
        425                 430                 435

TTC GCC GAG GAC AGC AAG AGC ATA GCC ACC GAG ATC ATC CTG GAG ATT     1399
Phe Ala Glu Asp Ser Lys Ser Ile Ala Thr Glu Ile Ile Leu Glu Ile
    440                 445                 450

AAG AAG GCA TTT GAG GAA AGC CTG AGC ACC CTG AAG TGG ATG GAT GAG     1447
Lys Lys Ala Phe Glu Glu Ser Leu Ser Thr Leu Lys Trp Met Asp Glu
455                 460                 465                 470

GAA ACC CGA AAA TCA GCC AAG GAA AAG GCC GAT GCC ATC TAC AAC ATG     1495
Glu Thr Arg Lys Ser Ala Lys Glu Lys Ala Asp Ala Ile Tyr Asn Met
                475                 480                 485

ATA GGA TAC CCC AAC TTC ATC ATG GAT CCC AAG GAG CTG GAC AAA GTG     1543
Ile Gly Tyr Pro Asn Phe Ile Met Asp Pro Lys Glu Leu Asp Lys Val
            490                 495                 500

TTT AAT GAC TAC ACT GCA GTT CCA GAC CTC TAC TTT GAA AAT GCC ATG     1591
Phe Asn Asp Tyr Thr Ala Val Pro Asp Leu Tyr Phe Glu Asn Ala Met
        505                 510                 515

CGG TTT TTC AAC TTC TCA TGG AGG GTC ACT GCC GAT CAG CTC AGG AAA     1639
Arg Phe Phe Asn Phe Ser Trp Arg Val Thr Ala Asp Gln Leu Arg Lys
    520                 525                 530
```

GCC CCC AAC AGA GAT CAG TGG AGC ATG ACC CCG CCC ATG GTG AAC GCC 1687
Ala Pro Asn Arg Asp Gln Trp Ser Met Thr Pro Pro Met Val Asn Ala
535 540 545 550

TAC TAC TCG CCC ACC AAG AAT GAG ATT GTG TTT CCG GCC GGG ATC CTG 1735
Tyr Tyr Ser Pro Thr Lys Asn Glu Ile Val Phe Pro Ala Gly Ile Leu
555 560 565

CAG GCA CCA TTC TAC ACA CGC TCC TCA CCC AAG GCC TTA AAC TTT GGT 1783
Gln Ala Pro Phe Tyr Thr Arg Ser Ser Pro Lys Ala Leu Asn Phe Gly
570 575 580

GGC ATA GGT GTC GTC GTG GGC CAT GAG CTG ACT CAT GCT TTT GAT GAT 1831
Gly Ile Gly Val Val Val Gly His Glu Leu Thr His Ala Phe Asp Asp
585 590 595

CAA GGA CGG GAG TAT GAC AAG GAC GGG AAC CTC CGG CCA TGG TGG AAG 1879
Gln Gly Arg Glu Tyr Asp Lys Asp Gly Asn Leu Arg Pro Trp Trp Lys
600 605 610

AAC TCA TCC GTG GAG GCC TTC AAG CGT CAG ACC GAG TGC ATG GTA GAG 1927
Asn Ser Ser Val Glu Ala Phe Lys Arg Gln Thr Glu Cys Met Val Glu
615 620 625 630

CAG TAC AGC AAC TAC AGC GTG AAC GGG GAG CCG GTG AAC GGG CGG CAC 1975
Gln Tyr Ser Asn Tyr Ser Val Asn Gly Glu Pro Val Asn Gly Arg His
635 640 645

ACC CTG GGG GAG AAC ATC GCC GAC AAC GGG GGT CTC AAG GCG GCC TAT 2023
Thr Leu Gly Glu Asn Ile Ala Asp Asn Gly Gly Leu Lys Ala Ala Tyr
650 655 660

CGG GCT TAC CAG AAC TGG GTG AAG AAG AAC GGG GCT GAG CAC TCG CTC 2071
Arg Ala Tyr Gln Asn Trp Val Lys Lys Asn Gly Ala Glu His Ser Leu
665 670 675

CCC ACC CTG GGC CTC ACC AAT AAC CAG CTC TTC TTC CTG GGC TTT GCA 2119
Pro Thr Leu Gly Leu Thr Asn Asn Gln Leu Phe Phe Leu Gly Phe Ala
680 685 690

CAG GTC TGG TGC TCC GTC CGC ACA CCT GAG AGC TCC CAC GAA GGC CTC 2167
Gln Val Trp Cys Ser Val Arg Thr Pro Glu Ser Ser His Glu Gly Leu
695 700 705 710

ATC ACC GAT CCC CAC AGC CCC TCT CGC TTC CGG GTC ATC GGC TCC CTC 2215
Ile Thr Asp Pro His Ser Pro Ser Arg Phe Arg Val Ile Gly Ser Leu
715 720 725

TCC AAT TCC AAG GAG TTC TCA GAA CAC TTC CGC TGC CCA CCT GGC TCA 2263
Ser Asn Ser Lys Glu Phe Ser Glu His Phe Arg Cys Pro Pro Gly Ser
730 735 740

```
CCC ATG AAC CCG CCT CAC AAG TGC GAA GTC TGG T AAGGACGAAG           2307
Pro Met Asn Pro Pro His Lys Cys Glu Val Trp
        745                 750

CGGAGAGAGC CAAGACGGAG GAGGGGAAGG GGCTGAGGAC GAGACCCCCA TCCAGCCTCC   2367

AGGGCATTGC TCAGCCCGCT TGGCCACCCG GGGCCCTGCT TCCTCACACT GGCGGGTTTT   2427

CAGCCGGAAC CGAGCCCATG GTGTTGGCTC TCAACGTGAC CCGCAGTCTG ATCCCCTGTG   2487

AAGAGCCGGA CATCCCAGGC ACACGTGTGC GCCACCTTCA GCAGGCATTC GGGTGCTGGG   2547

CTGGTGGCTC ATCAGGCCTG GGCCCCACAC TGACAAGCGC CAGATACGCC ACAAATACCA   2607

CTGTGTCAAA TGCTTTCAAG ATATATTTTT GGGGAAACTA TTTTTTAAAC ACTGTGGAAT   2667

ACACTGGAAA TCTTCAGGGA AAAACACATT TAAACACTTT TTTTTTTAAG CCC          2720
```

(2) INFORMATION ZU SEQ ID NO: 36:

(i) SEQUENZ CHARAKTERISTIKA:

(A) LÄNGE: 753 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 36:

Met Ser Thr Tyr Lys Arg Ala Thr Leu Asp Glu Glu Asp Leu Val Asp
1               5                   10                  15

Ser Leu Ser Glu Gly Asp Ala Tyr Pro Asn Gly Leu Gln Val Asn Phe
            20                  25                  30

His Ser Pro Arg Ser Gly Gln Arg Cys Trp Ala Ala Arg Thr Gln Val
        35                  40                  45

Glu Lys Arg Leu Val Val Leu Val Val Leu Leu Ala Ala Gly Leu Val
    50                  55                  60

Ala Cys Leu Ala Ala Leu Gly Ile Gln Tyr Gln Thr Arg Ser Pro Ser
65                  70                  75                  80

Val Cys Leu Ser Glu Ala Cys Val Ser Val Thr Ser Ser Ile Leu Ser
                85                  90                  95

Ser Met Asp Pro Thr Val Asp Pro Cys His Asp Phe Phe Ser Tyr Ala
            100                 105                 110

Cys Gly Gly Trp Ile Lys Ala Asn Pro Val Pro Asp Gly His Ser Arg
        115                 120                 125

Trp Gly Thr Phe Ser Asn Leu Trp Glu His Asn Gln Ala Ile Ile Lys
    130                 135                 140

His Leu Leu Glu Asn Ser Thr Ala Ser Val Ser Glu Ala Glu Arg Lys
145 150 155 160

Ala Gln Val Tyr Tyr Arg Ala Cys Met Asn Glu Thr Arg Ile Glu Glu
165 170 175

Leu Arg Ala Lys Pro Leu Met Glu Leu Ile Glu Arg Leu Gly Gly Trp
180 185 190

Asn Ile Thr Gly Pro Trp Ala Lys Asp Asn Phe Gln Asp Thr Leu Gln
195 200 205

Val Val Thr Ala His Tyr Arg Thr Ser Pro Phe Phe Ser Val Tyr Val
210 215 220

Ser Ala Asp Ser Lys Asn Ser Asn Ser Asn Val Ile Gln Val Asp Gln
225 230 235 240

Ser Gly Leu Gly Leu Pro Ser Arg Asp Tyr Tyr Leu Asn Lys Thr Glu
245 250 255

Asn Glu Lys Val Leu Thr Gly Tyr Leu Asn Tyr Met Val Gln Leu Gly
260 265 270

Lys Leu Leu Gly Gly Gly Asp Glu Glu Ala Ile Arg Pro Gln Met Gln
275 280 285

Gln Ile Leu Asp Phe Glu Thr Ala Leu Ala Asn Ile Thr Ile Pro Gln
290 295 300

Glu Lys Arg Arg Asp Glu Glu Leu Ile Tyr His Lys Val Thr Ala Ala
305 310 315 320

Glu Leu Gln Thr Leu Ala Pro Ala Ile Asn Trp Leu Pro Phe Leu Asn
325 330 335

Thr Ile Phe Tyr Pro Val Glu Ile Asn Glu Ser Glu Pro Ile Val Val
340 345 350

Tyr Asp Lys Glu Tyr Leu Glu Gln Ile Ser Thr Leu Ile Asn Thr Thr
355 360 365

Asp Arg Cys Leu Leu Asn Asn Tyr Met Ile Trp Asn Leu Val Arg Lys
370 375 380

Thr Ser Ser Phe Leu Asp Gln Arg Phe Gln Asp Ala Asp Glu Lys Phe
385 390 395 400

Met Glu Val Met Tyr Gly Thr Lys Lys Thr Cys Leu Pro Arg Trp Lys
405 410 415

Phe Cys Val Ser Asp Thr Glu Asn Asn Leu Gly Phe Ala Leu Gly Pro
420 425 430

Met Phe Val Lys Ala Thr Phe Ala Glu Asp Ser Lys Ser Ile Ala Thr
435 440 445

Glu Ile Ile Leu Glu Ile Lys Lys Ala Phe Glu Glu Ser Leu Ser Thr
450 455 460

Leu Lys Trp Met Asp Glu Glu Thr Arg Lys Ser Ala Lys Glu Lys Ala
465 470 475 480

Asp Ala Ile Tyr Asn Met Ile Gly Tyr Pro Asn Phe Ile Met Asp Pro
485 490 495

Lys Glu Leu Asp Lys Val Phe Asn Asp Tyr Thr Ala Val Pro Asp Leu
500 505 510

Tyr Phe Glu Asn Ala Met Arg Phe Phe Asn Phe Ser Trp Arg Val Thr
515 520 525

Ala Asp Gln Leu Arg Lys Ala Pro Asn Arg Asp Gln Trp Ser Met Thr
530 535 540

Pro Pro Met Val Asn Ala Tyr Tyr Ser Pro Thr Lys Asn Glu Ile Val
545 550 555 560

Phe Pro Ala Gly Ile Leu Gln Ala Pro Phe Tyr Thr Arg Ser Ser Pro
565 570 575

Lys Ala Leu Asn Phe Gly Gly Ile Gly Val Val Val Gly His Glu Leu
580 585 590

Thr His Ala Phe Asp Asp Gln Gly Arg Glu Tyr Asp Lys Asp Gly Asn
595 600 605

Leu Arg Pro Trp Trp Lys Asn Ser Ser Val Glu Ala Phe Lys Arg Gln
610 615 620

Thr Glu Cys Met Val Glu Gln Tyr Ser Asn Tyr Ser Val Asn Gly Glu
625 630 635 640

Pro Val Asn Gly Arg His Thr Leu Gly Glu Asn Ile Ala Asp Asn Gly
645 650 655

Gly Leu Lys Ala Ala Tyr Arg Ala Tyr Gln Asn Trp Val Lys Lys Asn
660 665 670

Gly Ala Glu His Ser Leu Pro Thr Leu Gly Leu Thr Asn Asn Gln Leu
675 680 685

Phe Phe Leu Gly Phe Ala Gln Val Trp Cys Ser Val Arg Thr Pro Glu
690 695 700

Ser Ser His Glu Gly Leu Ile Thr Asp Pro His Ser Pro Ser Arg Phe
705 710 715 720

```
Arg Val Ile Gly Ser Leu Ser Asn Ser Lys Glu Phe Ser Glu His Phe
                725                 730                 735

Arg Cys Pro Pro Gly Ser Pro Met Asn Pro Pro His Lys Cys Glu Val
            740                 745                 750

Trp
```